# EUROPEAN PATENT APPLICATION

(11) **EP 1 728 868 A2**
(43) Date of publication of application: **06.12.2006**
(21) Application number: 06000993.3
(22) Date of filing: 31.10.1997
(51) Int. Cl.: C12N 15/82, C12N 15/53, C12N 15/54, C12N 15/55, A01H 5/00

(54) **Herbicide resistant plants**

(30) Priority: 07.11.1996 GB 9623248; 13.12.1996 GB 9625957; 25.02.1997 GB 9703855
(62) Divisional of application: 97910550.9
(71) Applicant: Syngenta Limited, Guildford Surrey GU2 7YH (GB)
(72) Inventor: Hawkes, Timothy Robert, c/o Syngenta Limited, Bracknell, Berks RG42 6YA (GB); Jepson, Ian, c/o Syngenta Limited, Bracknell, Berks RG42 6YA (GB); Knight, Mary Elizabeth, c/o Syngenta Limited, Bracknell, Berks RG42 6YA (GB); Thomas, Paul Graham, c/o Syngenta Limited, Bracknell, Berks RG42 6YA (GB); Thompson, Paul Anthony, c/o Syngenta Limited, Bracknell, Berks RG42 6YA (GB)
(74) Representative: Gaal, Jozsef Christopher

(57) **Abstract**

The present invention provides, *inter alia,* a polynucleotide comprising at least a first region encoding a first protein capable of conferring on a plant, or tissue comprising it, resistance or tolerance to a first herbicide, and a second region encoding a second protein likewise capable of conferring resistance to a second herbicide, with the *provisos* (i) that the polynucleotide does not encode a fusion protein comprising only a 5-enol-pyruvyl-3-phosphoshikimate synthetase (EPSPS) and a glutathione S transferase (GST); (ii) that the polynucleotide does not comprise only regions encoding superoxide dismutase (SOD) and glutathione S transferase (GST); and (iii) that the polynucleotide does not comprise only regions encoding GST and phosphinothricin acetyl transferase (PAT).

SEQUENCE LISTING

(1) GENERAL INFORMATION:

(i) APPLICANT:
(A) NAME: SYNGENTA LIMITED
(B) STREET: European Regional Centre, Priestley Road,
(C) CITY: Surrey Research Park, Guildford
(E) COUNTRY: England
(F) POSTAL CODE (ZIP): GU2 7YH
(G) TELEPHONE: 01344 414521

(ii) TITLE OF INVENTION: Herbicide resistant plants

(iii) NUMBER OF SEQUENCES: 32

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 1020 base pairs
(B) TYPE: nucleic acid
(C) STRANDEDNESS: unknown
(D) TOPOLOGY: unknown

(ii) MOLECULE TYPE: DNA

(iii) HYPOTHETICAL: NO

(iv) ANTI-SENSE: NO

(vi) ORIGINAL SOURCE:
(A) ORGANISM: Synechocystis sp. PCC6803

(ix) FEATURE:
(A) NAME/KEY: CDS
(B) LOCATION:1..1020

(xi) SEQUENCE DESCRIPTION: SEQ ID NO: 1:

ATG GAA TTC GAC TAT CTT CAT TTA TAC GTT GAC GAT TAT CAG TCA GCT 48
Met Glu Phe Asp Tyr Leu His Leu Tyr Val Asp Asp Tyr Gln Ser Ala
1               5                   10                  15

CAT CGT TGT TAT CAA CGT CAA TGG GGT TTC ACT TGC GTA AAT AAA ATT 96
His Arg Cys Tyr Gln Arg Gln Trp Gly Phe Thr Cys Val Asn Lys Ile
            20                  25                  30

ATT ACT GAC CAA GGA ATT ACT GGC ATC TAC CAA CAG GGG CAA ATA CTT 144
Ile Thr Asp Gln Gly Ile Thr Gly Ile Tyr Gln Gln Gly Gln Ile Leu
        35                  40                  45

CTG CTA ATT TCG GCA TCG GAA TCT AGT TTG AGT AGA TAT GCC GAC TAT 192
Leu Leu Ile Ser Ala Ser Glu Ser Ser Leu Ser Arg Tyr Ala Asp Tyr
    50                  55                  60

CTC CAG AAA CAT CCC CCC GGC GTA GGT GAA GTC GCT TGG CAG GTG GCC 240
Leu Gln Lys His Pro Pro Gly Val Gly Glu Val Ala Trp Gln Val Ala
65                  70                  75                  80

AAT TGG CAA AAA ATT CAG CAT CAA TTA TCA GAA TTA CAG ATA GAA ACC 288
Asn Trp Gln Lys Ile Gln His Gln Leu Ser Glu Leu Gln Ile Glu Thr
                85                  90                  95

ACA CCA GTT ATT CAT CCT CTG ACT AAA GCA GAA GGA TTA ACT TTT TTG 336
Thr Pro Val Ile His Pro Leu Thr Lys Ala Glu Gly Leu Thr Phe Leu
            100                 105                 110

CTC TGG GGA GAT GTG CAC CAT AGC ATT TAT CCT GTT CGT TCT GAG CTA 384
Leu Trp Gly Asp Val His His Ser Ile Tyr Pro Val Arg Ser Glu Leu
        115                 120                 125

AAT CAG AAT AAA ACA TTG CAT GGT GTT GGT TTA ACG ACC ATC GAC CAT 432
Asn Gln Asn Lys Thr Leu His Gly Val Gly Leu Thr Thr Ile Asp His
    130                 135                 140

GTG GTG CTA AAC ATT GCC GCC GAT CAA TTT ACC CAG GCT TCC CAA TGG 480
Val Val Leu Asn Ile Ala Ala Asp Gln Phe Thr Gln Ala Ser Gln Trp
145                 150                 155                 160

TAT CAA CAG GTG TTT GGC TGG TCG GTG CAG CAG AGT TTT ACT GTC AAT 528
Tyr Gln Gln Val Phe Gly Trp Ser Val Gln Gln Ser Phe Thr Val Asn
                165                 170                 175

ACG CCC CAT TCT GGT CTG TAT AGC GAA GCC CTG GCC AGT GCC AAT GGG 576
Thr Pro His Ser Gly Leu Tyr Ser Glu Ala Leu Ala Ser Ala Asn Gly
            180                 185                 190

AAA GTC CAA TTT AAC CTC AAT TGT CCC ACC AAT AAC AGT TCC CAA ATT 624
Lys Val Gln Phe Asn Leu Asn Cys Pro Thr Asn Asn Ser Ser Gln Ile
        195                 200                 205

CAA ACT TTT TTA GCC AAT AAC CAT GGG GCT GGT ATT CAA CAT GTC GCT 672
Gln Thr Phe Leu Ala Asn Asn His Gly Ala Gly Ile Gln His Val Ala
    210                 215                 220

TTT TCC ACT ACG AGT ATT ACG CGA ACT GTG GCT CAT CTG CGG GAA AGG 720
Phe Ser Thr Thr Ser Ile Thr Arg Thr Val Ala His Leu Arg Glu Arg
225                 230                 235                 240

GGC GTA AAT TTT TTA AAA ATC CCC ACT GGC TAT TAT CAA CAG CAA AGA 768
Gly Val Asn Phe Leu Lys Ile Pro Thr Gly Tyr Tyr Gln Gln Gln Arg
                245                 250                 255

AAC AGT AGC TAT TTT AAT TAT GCA AGT TTG GAT TGG GAT ACC TTA CAG 816
Asn Ser Ser Tyr Phe Asn Tyr Ala Ser Leu Asp Trp Asp Thr Leu Gln
            260                 265                 270

TGC CTA GAA ATT TTG CTG GAT GAT CAA GAT AAT ACG GGG GAG CGA TTA 864
Cys Leu Glu Ile Leu Leu Asp Asp Gln Asp Asn Thr Gly Glu Arg Leu
        275                 280                 285

CTG CTA CAA ATT TTT AGT CAG CCT TGC TAT GGA GTA GGC ACT CTA TTT 912
Leu Leu Gln Ile Phe Ser Gln Pro Cys Tyr Gly Val Gly Thr Leu Phe
    290                 295                 300

TGG GAA ATT ATT GAA CGC CGC CAC CGG GCA AAA GGA TTT GGT CAA GGA 960
Trp Glu Ile Ile Glu Arg Arg His Arg Ala Lys Gly Phe Gly Gln Gly
305                 310                 315                 320

AAC TTT CAA GCT CTC TAT GAA GCG GTG GAG ACT TTA GAA AAA CAG TTA 1008
Asn Phe Gln Ala Leu Tyr Glu Ala Val Glu Thr Leu Glu Lys Gln Leu
                325                 330                 335

GAA GTG CCA TAA 1020
Glu Val Pro

(2) INFORMATION FOR SEQ ID NO: 2:

(i) SEQUENCE CHARACTERISTICS:
(A) LENGTH: 339 amino acids
(B) TYPE: amino acid
(D) TOPOLOGY: linear

(ii) MOLECULE TYPE: protein
(xi) SEQUENCE DESCRIPTION: SEQ ID NO: 2:

Met Glu Phe Asp Tyr Leu His Leu Tyr Val Asp Asp Tyr Gln Ser Ala
1               5                   10                  15

His Arg Cys Tyr Gln Arg Gln Trp Gly Phe Thr Cys Val Asn Lys Ile
            20                  25                  30

Ile Thr Asp Gln Gly Ile Thr Gly Ile Tyr Gln Gln Gly Gln Ile Leu
        35                  40                  45

Leu Leu Ile Ser Ala Ser Glu Ser Ser Leu Ser Arg Tyr Ala Asp Tyr
    50                  55                  60

Leu Gln Lys His Pro Pro Gly Val Gly Glu Val Ala Trp Gln Val Ala
65                  70                  75                  80

Asn Trp Gln Lys Ile Gln His Gln Leu Ser Glu Leu Gln Ile Glu Thr
                85                  90                  95

Thr Pro Val Ile His Pro Leu Thr Lys Ala Glu Gly Leu Thr Phe Leu
            100                 105                 110

Leu Trp Gly Asp Val His His Ser Ile Tyr Pro Val Arg Ser Glu Leu
        115                 120                 125

Asn Gln Asn Lys Thr Leu His Gly Val Gly Leu Thr Thr Ile Asp His
    130                 135                 140

Val Val Leu Asn Ile Ala Ala Asp Gln Phe Thr Gln Ala Ser Gln Trp
145                 150                 155                 160

Tyr Gln Gln Val Phe Gly Trp Ser Val Gln Gln Ser Phe Thr Val Asn
                165                 170                 175

Thr Pro His Ser Gly Leu Tyr Ser Glu Ala Leu Ala Ser Ala Asn Gly
            180                 185                 190

Lys Val Gln Phe Asn Leu Asn Cys Pro Thr Asn Asn Ser Ser Gln Ile
        195                 200                 205

Gln Thr Phe Leu Ala Asn Asn His Gly Ala Gly Ile Gln His Val Ala
    210                 215                 220

Phe Ser Thr Thr Ser Ile Thr Arg Thr Val Ala His Leu Arg Glu Arg
225                 230                 235                 240

Gly Val Asn Phe Leu Lys Ile Pro Thr Gly Tyr Tyr Gln Gln Gln Arg
                245                 250                 255

Asn Ser Ser Tyr Phe Asn Tyr Ala Ser Leu Asp Trp Asp Thr Leu Gln
            260                 265                 270

Cys Leu Glu Ile Leu Leu Asp Asp Gln Asp Asn Thr Gly Glu Arg Leu
        275                 280                 285

Leu Leu Gln Ile Phe Ser Gln Pro Cys Tyr Gly Val Gly Thr Leu Phe
    290                 295                 300

Trp Glu Ile Ile Glu Arg Arg His Arg Ala Lys Gly Phe Gly Gln Gly
305                 310                 315                 320

Asn Phe Gln Ala Leu Tyr Glu Ala Val Glu Thr Leu Glu Lys Gln Leu
                325                 330                 335

Glu Val Pro

(2) INFORMATION FOR SEQ ID NO: 3:

     (i) SEQUENCE CHARACTERISTICS:
          (A) LENGTH: 2582 base pairs
          (B) TYPE: nucleic acid
          (C) STRANDEDNESS: single
          (D) TOPOLOGY: linear

    (ii) MOLECULE TYPE: cDNA

   (iii) HYPOTHETICAL: NO

    (iv) ANTI-SENSE: NO

    (vi) ORIGINAL SOURCE:
          (A) ORGANISM: Pseudomonas fluorescens

    (ix) FEATURE:
          (A) NAME/KEY: CDS
          (B) LOCATION:1217..2290

    (xi) SEQUENCE DESCRIPTION: SEQ ID NO: 3:

ATTAGTCGAA GAATATGCCC ATCCTGTCGC CTGTCGAGCA ACTGCTAATG CAACCTCCGT      60

CTGATCGCCT CACCTACCTG AAGCTGGCCG CTGTGACCAT GATTTGGGGT GGCACTTTTG     120

TCGCCGGACG TTACCTGACC AATCAAGTCG ACCCGCTGCT GGCCGCCAGC CTGCGGTTTA     180

TCCTGGCCAG CCTGGCGCTG CTGCTGTTTA TGCTGTGTGC ACGCATCCCG CTGGCGCGGC     240

CACGTCCCCG GCAACTGCTG CATCTGGCGG TGCTGGGGTT TTTCGGGATC TTTTTCTACA     300

ACCTGTGTTT TTTCTACGGC CTGCAGTACA TCAACGCCTC GCGCGCTTCG TTGATCGTGG     360

CGTTGAATCC GGCGGTGATC GGCCTGGCTT CCTGGTGGTT GTTCAAAGAG CGCCTCGGCA     420

CTGCCAGGGT GCTGGGTATC GCGTTGTGCC TGGCCGGCGC TGCGACGGTG ATCGTCAGTC     480

GCAACCCGCA GTTGCTGCAA GGTGCATCGA GTACCTGGCA GGGCGACCTG CTGGTGTTCG     540

GCTGTGTGCT GGGGTGGGGG ATTTACTCGT TGTTTTCCCG CGCATTGAAT CAAAGCCTGG     600

GGCCGTTGCA AACGGTCACC TGGTCAGTGC TGCTGGGCAC CCTGATGCTG ACGGCTGTCA     660

CCGCGCTCGC CGGGCGCTTC ACGCTTGCAG GGCTTGGCAG CCTGCACCTG CCGCAGGTTG 720

TGAGCCTGTT GTATTTGGGC GTGCTCGGCT CCGCGCTGGC GTACATCGGC TATTACGATG 780

GCATCCGGCG TATCGGCGCG ACCCGCGCAG GCGTGTTTAT CGCGCTGAAC CCGCTGACGG 840

CGGTGATCTG CGGCGCGCTG CTGCTTGGCG AACAGCTAAC GTTACCCATG GCGCTCGGCG 900

GCGCGGTGAT CCTGTTGGGC ATCTATCTGT GCAACAAACC CCTTGCGCAG CCCAGCGCAA 960

TAGGGATTTG ATGAGAGTGC GGACAAATAC TGTTACGCTG TGTAGAATCG ATTTACGCAT 1020

ACAAGAATAT GGACTTGCGC TCACGCAAGC CTCGGCCGTC AGAGACTGAT GTAATCATGA 1080

AGCTACTCGG CTCCCCCCTG ATCTTTGGTG ACTTCCTCGC GCGCAGCGTG CGGGGTCTCT 1140

CGTGCGCGCC ACCCTGCAAC CTCATCCTTG CCTGTAATTG ACTGCTTGCT ACTTACAAGA 1200

ATGATGAGGT GCCGAA ATG GCC GAC CAA TAC GAA AAC CCA ATG GGC CTG 1249
Met Ala Asp Gln Tyr Glu Asn Pro Met Gly Leu
1               5                   10

ATG GGC TTT GAA TTT ATT GAA TTC GCA TCG CCG ACT CCG GGC ACC CTG 1297
Met Gly Phe Glu Phe Ile Glu Phe Ala Ser Pro Thr Pro Gly Thr Leu
            15                  20                  25

GAG CCG ATC TTC GAG ATC ATG GGC TTC ACC AAA GTC GCG ACC CAC CGC 1345
Glu Pro Ile Phe Glu Ile Met Gly Phe Thr Lys Val Ala Thr His Arg
        30                  35                  40

TCC AAG AAT GTG CAC CTG TAC CGC CAG GGC GAG ATC AAC CTG ATC CTC 1393
Ser Lys Asn Val His Leu Tyr Arg Gln Gly Glu Ile Asn Leu Ile Leu
    45                  50                  55

AAC AAC CAG CCC GAC AGC CTG GCC TCG TAC TTC GCC GCC GAA CAC GGC 1441
Asn Asn Gln Pro Asp Ser Leu Ala Ser Tyr Phe Ala Ala Glu His Gly
60                  65                  70                  75

CCT TCG GTG TGC GGC ATG GCG TTC CGG GTC AAA GAC TCG CAG CAG GCT 1489
Pro Ser Val Cys Gly Met Ala Phe Arg Val Lys Asp Ser Gln Gln Ala
                80                  85                  90

TAC AAC CGC GCG TTG GAA CTG GGC GCC CAG CCG ATT CAT ATC GAA ACC 1537
Tyr Asn Arg Ala Leu Glu Leu Gly Ala Gln Pro Ile His Ile Glu Thr
            95                  100                 105

GGC CCG ATG GAA CTC AAC CTG CCG GCC ATC AAG GGC ATC GGC GGT GCG 1585
Gly Pro Met Glu Leu Asn Leu Pro Ala Ile Lys Gly Ile Gly Gly Ala
        110                 115                 120

CCG CTG TAC CTG ATC GAC CGC TTC GGT GAA GGC AGC TCG ATA TAT GAC 1633
Pro Leu Tyr Leu Ile Asp Arg Phe Gly Glu Gly Ser Ser Ile Tyr Asp
    125                 130                 135

ATC GAC TTC GTG TAC CTC GAA GGT GTC GAC CGC AAC CCG GTA GGC GCG 1681
Ile Asp Phe Val Tyr Leu Glu Gly Val Asp Arg Asn Pro Val Gly Ala
140                 145                 150                 155

GGC CTC AAG GTC ATC GAC CAC CTG ACC CAC AAC GTG TAT CGC GGC CGC 1729
Gly Leu Lys Val Ile Asp His Leu Thr His Asn Val Tyr Arg Gly Arg
                160                 165                 170

ATG GCC TAC TGG GCC AAC TTC TAC GAG AAA CTG TTC AAC TTC CGT GAA 1777

Met Ala Tyr Trp Ala Asn Phe Tyr Glu Lys Leu Phe Asn Phe Arg Glu
            175                 180                 185

GCA CGC TAC TTC GAT ATC AAG GGC GAA TAC ACC GGC CTT ACG TCC AAG 1825
Ala Arg Tyr Phe Asp Ile Lys Gly Glu Tyr Thr Gly Leu Thr Ser Lys
        190                 195                 200

GCC ATG AGT GCC CCG GAC GGC ATG ATC CGC ATC CCG CTG AAC GAG GAA 1873
Ala Met Ser Ala Pro Asp Gly Met Ile Arg Ile Pro Leu Asn Glu Glu
    205                 210                 215

TCG TCC AAG GGC GCC GGC CAG ATC GAA GAG TTC CTG ATG CAG TTC AAC 1921
Ser Ser Lys Gly Ala Gly Gln Ile Glu Glu Phe Leu Met Gln Phe Asn
220                 225                 230                 235

GGC GAG GGC ATC CAG CAC GTG GCG TTC CTC ACC GAA GAC CTG GTC AAG 1969
Gly Glu Gly Ile Gln His Val Ala Phe Leu Thr Glu Asp Leu Val Lys
                240                 245                 250

ACC TGG GAT GCG TTG AAG AAG ATC GGC ATG CGC TTC ATG ACC GCG CCG 2017
Thr Trp Asp Ala Leu Lys Lys Ile Gly Met Arg Phe Met Thr Ala Pro
            255                 260                 265

CCG GAC ACC TAC TAC GAA ATG CTC GAA GGC CGC CTG CCA AAC CAC GGC 2065
Pro Asp Thr Tyr Tyr Glu Met Leu Glu Gly Arg Leu Pro Asn His Gly
        270                 275                 280

GAG CCG GTG GAC CAA CTG CAG GCG CGC GGT ATT TTG CTG GAC GGC TCC 2113
Glu Pro Val Asp Gln Leu Gln Ala Arg Gly Ile Leu Leu Asp Gly Ser
    285                 290                 295

TCG ATC GAG GGC GAC AAG CGC CTG CTG CTG CAG ATC TTC TCG GAA ACC 2161
Ser Ile Glu Gly Asp Lys Arg Leu Leu Leu Gln Ile Phe Ser Glu Thr
300                 305                 310                 315

CTG ATG GGC CCG GTG TTC TTC GAA TTC ATC CAG CGC AAA GGC GAC GAT 2209
Leu Met Gly Pro Val Phe Phe Glu Phe Ile Gln Arg Lys Gly Asp Asp
                320                 325                 330

GGG TTT GGC GAG GGC AAC TTC AAG GCG CTG TTC GAG TCG ATC GAG CGC 2257
Gly Phe Gly Glu Gly Asn Phe Lys Ala Leu Phe Glu Ser Ile Glu Arg
            335                 340                 345

GAC CAG GTA CGT CGC GGT GTA CTG ACC ACC GAC TAAGCGTCAG CAACAAAAAA 2310
Asp Gln Val Arg Arg Gly Val Leu Thr Thr Asp
        350                 355

AGCCCGGCGA GAAGGTTTTC AGCCGGGCTT TTTAGTGCCT GCACGTTTTA AGCTTTGCGC 2370

TGACGCACCA AATGTTTGAA GCCTTCATAC ACCAGCACCA TCACGGCCAG CCAGATCGGG 2430

ATATACGTCA GCCATTGCCC GCCCTTGATG CCTTCGCCCA ACAACAAGGC CACAAAGAGC 2490

AGTAATACCG GCTCCACATA GCTGAGCAAC CCGAACAGGC TAAAGGCCAA TAAACGGCTG 2550

GCGATGATGT AGCTCACCAG CGCTGAAGCA CT 2582

(2) INFORMATION FOR SEQ ID NO: 4:

(i) SEQUENCE CHARACTERISTICS:
(A) LENGTH: 358 amino acids
(B) TYPE: amino acid
(D) TOPOLOGY: linear

(ii) MOLECULE TYPE: protein
(xi) SEQUENCE DESCRIPTION: SEQ ID NO: 4:

Met Ala Asp Gln Tyr Glu Asn Pro Met Gly Leu Met Gly Phe Glu Phe
1               5                   10                  15

Ile Glu Phe Ala Ser Pro Thr Pro Gly Thr Leu Glu Pro Ile Phe Glu
            20                  25                  30

Ile Met Gly Phe Thr Lys Val Ala Thr His Arg Ser Lys Asn Val His
        35                  40                  45

Leu Tyr Arg Gln Gly Glu Ile Asn Leu Ile Leu Asn Asn Gln Pro Asp
    50                  55                  60

Ser Leu Ala Ser Tyr Phe Ala Ala Glu His Gly Pro Ser Val Cys Gly
65                  70                  75                  80

Met Ala Phe Arg Val Lys Asp Ser Gln Gln Ala Tyr Asn Arg Ala Leu
                85                  90                  95

Glu Leu Gly Ala Gln Pro Ile His Ile Glu Thr Gly Pro Met Glu Leu
            100                 105                 110

Asn Leu Pro Ala Ile Lys Gly Ile Gly Gly Ala Pro Leu Tyr Leu Ile
        115                 120                 125

Asp Arg Phe Gly Glu Gly Ser Ser Ile Tyr Asp Ile Asp Phe Val Tyr
    130                 135                 140

Leu Glu Gly Val Asp Arg Asn Pro Val Gly Ala Gly Leu Lys Val Ile
145                 150                 155                 160

Asp His Leu Thr His Asn Val Tyr Arg Gly Arg Met Ala Tyr Trp Ala
                165                 170                 175

Asn Phe Tyr Glu Lys Leu Phe Asn Phe Arg Glu Ala Arg Tyr Phe Asp
            180                 185                 190

Ile Lys Gly Glu Tyr Thr Gly Leu Thr Ser Lys Ala Met Ser Ala Pro
        195                 200                 205

Asp Gly Met Ile Arg Ile Pro Leu Asn Glu Glu Ser Ser Lys Gly Ala
    210                 215                 220

Gly Gln Ile Glu Glu Phe Leu Met Gln Phe Asn Gly Glu Gly Ile Gln
225                 230                 235                 240

His Val Ala Phe Leu Thr Glu Asp Leu Val Lys Thr Trp Asp Ala Leu
                245                 250                 255

Lys Lys Ile Gly Met Arg Phe Met Thr Ala Pro Pro Asp Thr Tyr Tyr
            260                 265                 270

Glu Met Leu Glu Gly Arg Leu Pro Asn His Gly Glu Pro Val Asp Gln
        275                 280                 285

Leu Gln Ala Arg Gly Ile Leu Leu Asp Gly Ser Ser Ile Glu Gly Asp
    290                 295                 300

Lys Arg Leu Leu Leu Gln Ile Phe Ser Glu Thr Leu Met Gly Pro Val
305                 310                 315                 320

Phe Phe Glu Phe Ile Gln Arg Lys Gly Asp Asp Gly Phe Gly Glu Gly

325                    330                    335

Asn Phe Lys Ala Leu Phe Glu Ser Ile Glu Arg Asp Gln Val Arg Arg
            340                    345                    350

Gly Val Leu Thr Thr Asp
        355

(2) INFORMATION FOR SEQ ID NO: 5:

(i) SEQUENCE CHARACTERISTICS:
(A) LENGTH: 17 base pairs
(B) TYPE: nucleic acid
(C) STRANDEDNESS: single
(D) TOPOLOGY: unknown

(ii) MOLECULE TYPE: other nucleic acid
(A) DESCRIPTION:   /desc = "primer"

(iii) HYPOTHETICAL: NO

(iv) ANTI-SENSE: NO

(vi) ORIGINAL SOURCE:
(A) ORGANISM: primer

(xi) SEQUENCE DESCRIPTION: SEQ ID NO: 5:

TATGAGAATC  CTATGGG                                                17

(2) INFORMATION FOR SEQ ID NO: 6:

(i) SEQUENCE CHARACTERISTICS:
(A) LENGTH: 17 base pairs
(B) TYPE: nucleic acid
(C) STRANDEDNESS: single
(D) TOPOLOGY: unknown

(ii) MOLECULE TYPE: other nucleic acid
(A) DESCRIPTION:   /desc = "primer"

(iii) HYPOTHETICAL: NO

(iv) ANTI-SENSE: NO

(vi) ORIGINAL SOURCE:
(A) ORGANISM: primer

(xi) SEQUENCE DESCRIPTION: SEQ ID NO: 6:

GCTTTGAA GTTTCCCTC

(2) INFORMATION FOR SEQ ID NO: 7:

(i) SEQUENCE CHARACTERISTICS:
(A) LENGTH: 46 base pairs
(B) TYPE: nucleic acid
(C) STRANDEDNESS: single
(D) TOPOLOGY: unknown

(ii) MOLECULE TYPE: other nucleic acid
(A) DESCRIPTION:   /desc = "primer"

(iii) HYPOTHETICAL: NO

(iv) ANTI-SENSE: NO

(vi) ORIGINAL SOURCE:
(A) ORGANISM: primer

(xi) SEQUENCE DESCRIPTION: SEQ ID NO: 7:

GTTAGGTACC AGTCTAGACT GACCATGGCC GACCAATACG AAAACC 46

(2) INFORMATION FOR SEQ ID NO: 8:

(i) SEQUENCE CHARACTERISTICS:
(A) LENGTH: 43 base pairs
(B) TYPE: nucleic acid
(C) STRANDEDNESS: single
(D) TOPOLOGY: unknown

(ii) MOLECULE TYPE: other nucleic acid
(A) DESCRIPTION: /desc = "primer"

(iii) HYPOTHETICAL: NO

(iv) ANTI-SENSE: NO

(vi) ORIGINAL SOURCE:
(A) ORGANISM: primer

(xi) SEQUENCE DESCRIPTION: SEQ ID NO: 8:

TAGCGGTACC TGATCACCCG GGTTATTAGT CGGTGGTCAG TAC 43

(2) INFORMATION FOR SEQ ID NO: 9:
(i) SEQUENCE CHARACTERISTICS:
(A) LENGTH: 516 amino acids
(B) TYPE: amino acid
(D) TOPOLOGY: linear

(ii) MOLECULE TYPE: protein
(xi) SEQUENCE DESCRIPTION: SEQ ID NO: 9:

Met Ala Gln Ile Asn Asn Met Ala Gln Gly Ile Gln Thr Leu Asn Pro
1               5                   10                  15

Asn Ser Asn Phe His Lys Pro Gln Val Pro Lys Ser Ser Ser Phe Leu
            20                  25                  30

Val Phe Gly Ser Lys Lys Leu Lys Asn Ser Ala Asn Ser Met Leu Val
        35                  40                  45

Leu Lys Lys Asp Ser Ile Phe Met Gln Lys Phe Cys Ser Phe Arg Ile
    50                  55                  60

Ser Ala Ser Val Ala Thr Ala Gln Lys Pro Ser Glu Ile Val Leu Gln
65                  70                  75                  80

Pro Ile Lys Glu Ile Ser Gly Thr Val Lys Leu Pro Gly Ser Lys Ser
                85                  90                  95

Leu Ser Asn Arg Ile Leu Leu Leu Ala Ala Leu Ser Glu Gly Thr Thr
            100                 105                 110

Val Val Asp Asn Leu Leu Ser Ser Asp Asp Ile His Tyr Met Leu Gly
        115                 120                 125

Ala Leu Lys Thr Leu Gly Leu His Val Glu Glu Asp Ser Ala Asn Gln
    130                 135                 140

Arg Ala Val Val Glu Gly Cys Gly Gly Leu Phe Pro Val Gly Lys Glu
145                 150                 155                 160

Ser Lys Glu Glu Ile Gln Leu Phe Leu Gly Asn Ala Gly Thr Ala Met
                165                 170                 175

Arg Pro Leu Thr Ala Ala Val Thr Val Ala Gly Gly Asn Ser Arg Tyr
            180                 185                 190

Val Leu Asp Gly Val Pro Arg Met Arg Glu Arg Pro Ile Ser Asp Leu
        195                 200                 205

Val Asp Gly Leu Lys Gln Leu Gly Ala Glu Val Asp Cys Phe Leu Gly
    210                 215                 220

Thr Lys Cys Pro Pro Val Arg Ile Val Ser Lys Gly Gly Leu Pro Gly
225                 230                 235                 240

Gly Lys Val Lys Leu Ser Gly Ser Ile Ser Ser Gln Tyr Leu Thr Ala
                245                 250                 255

Leu Leu Met Ala Ala Pro Leu Ala Leu Gly Asp Val Glu Ile Glu Ile
            260                 265                 270

Ile Asp Lys Leu Ile Ser Val Pro Tyr Val Glu Met Thr Leu Lys Leu
        275                 280                 285

Met Glu Arg Phe Gly Ile Ser Val Glu His Ser Ser Ser Trp Asp Arg
    290                 295                 300

Phe Phe Val Arg Gly Gly Gln Lys Tyr Lys Ser Pro Gly Lys Ala Phe
305                 310                 315                 320

Val Glu Gly Asp Ala Ser Ser Ala Ser Tyr Phe Leu Ala Gly Ala Ala
                325                 330                 335

Val Thr Gly Gly Thr Ile Thr Val Glu Gly Cys Gly Thr Asn Ser Leu
            340                 345                 350

Gln Gly Asp Val Lys Phe Ala Glu Val Leu Glu Lys Met Gly Ala Glu
        355                 360                 365

Val Thr Trp Thr Glu Asn Ser Val Thr Val Lys Gly Pro Pro Arg Ser
    370                 375                 380

Ser Ser Gly Arg Lys His Leu Arg Ala Ile Asp Val Asn Met Asn Lys
385                 390                 395                 400

Met Pro Asp Val Ala Met Thr Leu Ala Val Val Ala Leu Tyr Ala Asp
                405                 410                 415

Gly Pro Thr Ala Ile Arg Asp Val Ala Ser Trp Arg Val Lys Glu Thr
            420                 425                 430

Glu Arg Met Ile Ala Ile Cys Thr Glu Leu Arg Lys Leu Gly Ala Thr

435                 440                 445

Val Glu Glu Gly Pro Asp Tyr Cys Ile Ile Thr Pro Pro Glu Lys Leu
    450                 455                 460

Asn Val Thr Asp Ile Asp Thr Tyr Asp Asp His Arg Met Ala Met Ala
465                 470                 475                 480

Phe Ser Leu Ala Ala Cys Ala Asp Val Pro Val Thr Ile Asn Asp Pro
                485                 490                 495

Gly Cys Thr Arg Lys Thr Phe Pro Asn Tyr Phe Asp Val Leu Gln Gln
            500                 505                 510

Tyr Ser Lys His
        515

(2) INFORMATION FOR SEQ ID NO: 10:

(i) SEQUENCE CHARACTERISTICS:
(A) LENGTH: 17 base pairs
(B) TYPE: nucleic acid
(C) STRANDEDNESS: single
(D) TOPOLOGY: unknown

(ii) MOLECULE TYPE: other nucleic acid
(A) DESCRIPTION:   /desc = "primer"

(iii) HYPOTHETICAL: NO

(iv) ANTI-SENSE: NO

(vi) ORIGINAL SOURCE:
(A) ORGANISM: primer

(xi) SEQUENCE DESCRIPTION: SEQ ID NO: 10:

AACAAGGTGG   CGCAGTT                                                17

(2) INFORMATION FOR SEQ ID NO: 11:

(i) SEQUENCE CHARACTERISTICS:
(A) LENGTH: 20 base pairs
(B) TYPE: nucleic acid
(C) STRANDEDNESS: single
(D) TOPOLOGY: unknown

(ii) MOLECULE TYPE: other nucleic acid
(A) DESCRIPTION:   /desc = "primer"

(iii) HYPOTHETICAL: NO

(iv) ANTI-SENSE: NO

(vi) ORIGINAL SOURCE:
(A) ORGANISM: primer

(xi) SEQUENCE DESCRIPTION: SEQ ID NO: 11:

CATCGCAAGA   CCGGCAACAG                                             20

(2) INFORMATION FOR SEQ ID NO: 12:

(i) SEQUENCE CHARACTERISTICS:
(A) LENGTH: 19 base pairs
(B) TYPE: nucleic acid
(C) STRANDEDNESS: single
(D) TOPOLOGY: unknown

(ii) MOLECULE TYPE: other nucleic acid
(A) DESCRIPTION: /desc = "primer"

(iii) HYPOTHETICAL: NO

(iv) ANTI-SENSE: NO

(vi) ORIGINAL SOURCE:
(A) ORGANISM: primer

(xi) SEQUENCE DESCRIPTION: SEQ ID NO: 12:

GATCGCTACT AGCTTCCCA 19

(2) INFORMATION FOR SEQ ID NO: 13:

(i) SEQUENCE CHARACTERISTICS:
(A) LENGTH: 22 base pairs
(B) TYPE: nucleic acid
(C) STRANDEDNESS: single
(D) TOPOLOGY: unknown

(ii) MOLECULE TYPE: other nucleic acid
(A) DESCRIPTION: /desc = "primer"

(iii) HYPOTHETICAL: NO

(iv) ANTI-SENSE: NO

(vi) ORIGINAL SOURCE:
(A) ORGANISM: primer

(xi) SEQUENCE DESCRIPTION: SEQ ID NO: 13:

AATCAAGGTA ACCTTGAATC CA 22

(2) INFORMATION FOR SEQ ID NO: 14:

(i) SEQUENCE CHARACTERISTICS:
(A) LENGTH: 18 base pairs
(B) TYPE: nucleic acid
(C) STRANDEDNESS: single
(D) TOPOLOGY: unknown

(ii) MOLECULE TYPE: other nucleic acid
(A) DESCRIPTION: /desc = "primer"

(iii) HYPOTHETICAL: NO

(iv) ANTI-SENSE: NO

(vi) ORIGINAL SOURCE:

(A) ORGANISM: primer

(xi) SEQUENCE DESCRIPTION: SEQ ID NO: 14:

AATTACGGAA GCTTCCGT 18

(2) INFORMATION FOR SEQ ID NO: 15:

(i) SEQUENCE CHARACTERISTICS:
(A) LENGTH: 20 base pairs
(B) TYPE: nucleic acid
(C) STRANDEDNESS: single
(D) TOPOLOGY: unknown

(ii) MOLECULE TYPE: other nucleic acid
(A) DESCRIPTION: /desc = "primer"

(iii) HYPOTHETICAL: NO

(iv) ANTI-SENSE: NO

(vi) ORIGINAL SOURCE:
(A) ORGANISM: primer

(xi) SEQUENCE DESCRIPTION: SEQ ID NO: 15:

AGCTTGTACA CCGGTGTACA 20

(2) INFORMATION FOR SEQ ID NO: 16:

(i) SEQUENCE CHARACTERISTICS:
(A) LENGTH: 22 base pairs
(B) TYPE: nucleic acid
(C) STRANDEDNESS: single
(D) TOPOLOGY: unknown

(ii) MOLECULE TYPE: other nucleic acid
(A) DESCRIPTION: /desc = "primer"

(iii) HYPOTHETICAL: NO

(iv) ANTI-SENSE: NO

(vi) ORIGINAL SOURCE:
(A) ORGANISM: primer

(xi) SEQUENCE DESCRIPTION: SEQ ID NO: 16:

CGGACAATTA ATTGTCCGGT AC 22

(2) INFORMATION FOR SEQ ID NO: 17:

(i) SEQUENCE CHARACTERISTICS:
(A) LENGTH: 22 base pairs
(B) TYPE: nucleic acid
(C) STRANDEDNESS: single
(D) TOPOLOGY: unknown

(ii) MOLECULE TYPE: other nucleic acid

(A) DESCRIPTION: /desc = "primer"

(iii) HYPOTHETICAL: NO

(iv) ANTI-SENSE: NO

(vi) ORIGINAL SOURCE:
(A) ORGANISM: primer

(xi) SEQUENCE DESCRIPTION: SEQ ID NO: 17:

CATTTGCGGC CGCAAATGGT AC 22

(2) INFORMATION FOR SEQ ID NO: 18:

(i) SEQUENCE CHARACTERISTICS:
(A) LENGTH: 22 base pairs
(B) TYPE: nucleic acid
(C) STRANDEDNESS: single
(D) TOPOLOGY: unknown

(ii) MOLECULE TYPE: other nucleic acid
(A) DESCRIPTION: /desc = "primer"

(iii) HYPOTHETICAL: NO

(iv) ANTI-SENSE: NO

(vi) ORIGINAL SOURCE:
(A) ORGANISM: primer

(xi) SEQUENCE DESCRIPTION: SEQ ID NO: 18:

AATTCATTTG CGGCCGCAAA TG 22

(2) INFORMATION FOR SEQ ID NO: 19:

(i) SEQUENCE CHARACTERISTICS:
(A) LENGTH: 20 base pairs
(B) TYPE: nucleic acid
(C) STRANDEDNESS: single
(D) TOPOLOGY: unknown

(ii) MOLECULE TYPE: other nucleic acid
(A) DESCRIPTION: /desc = "primer"

(iii) HYPOTHETICAL: NO

(iv) ANTI-SENSE: NO

(vi) ORIGINAL SOURCE:
(A) ORGANISM: primer

(xi) SEQUENCE DESCRIPTION: SEQ ID NO: 19:

AGCTTGCAGC GGCCGCTGCA 20

(2) INFORMATION FOR SEQ ID NO: 20:

(i) SEQUENCE CHARACTERISTICS:

(A) LENGTH: 22 base pairs
(B) TYPE: nucleic acid
(C) STRANDEDNESS: single
(D) TOPOLOGY: unknown

(ii) MOLECULE TYPE: other nucleic acid
(A) DESCRIPTION: /desc = "primer"

(iii) HYPOTHETICAL: NO

(iv) ANTI-SENSE: NO

(vi) ORIGINAL SOURCE:
(A) ORGANISM: primer

(xi) SEQUENCE DESCRIPTION: SEQ ID NO: 20:

AATTCATTTG CGGCCGCAAA TG 22

(2) INFORMATION FOR SEQ ID NO: 21:

(i) SEQUENCE CHARACTERISTICS:
(A) LENGTH: 28 base pairs
(B) TYPE: nucleic acid
(C) STRANDEDNESS: single
(D) TOPOLOGY: unknown

(ii) MOLECULE TYPE: other nucleic acid
(A) DESCRIPTION: /desc = "primer"

(iii) HYPOTHETICAL: NO

(iv) ANTI-SENSE: NO

(vi) ORIGINAL SOURCE:
(A) ORGANISM: primer

(xi) SEQUENCE DESCRIPTION: SEQ ID NO: 21:

CTCGAGTATT TTTACAACAA TTACCAAC 28

(2) INFORMATION FOR SEQ ID NO: 22:

(i) SEQUENCE CHARACTERISTICS:
(A) LENGTH: 22 base pairs
(B) TYPE: nucleic acid
(C) STRANDEDNESS: single
(D) TOPOLOGY: unknown

(ii) MOLECULE TYPE: other nucleic acid
(A) DESCRIPTION: /desc = "primer"

(iii) HYPOTHETICAL: NO

(iv) ANTI-SENSE: NO

(vi) ORIGINAL SOURCE:
(A) ORGANISM: primer

(xi) SEQUENCE DESCRIPTION: SEQ ID NO: 22:

AATCAAGGTA ACCTTGAATC CA 22

(2) INFORMATION FOR SEQ ID NO: 23:

(i) SEQUENCE CHARACTERISTICS:
(A) LENGTH: 26 base pairs
(B) TYPE: nucleic acid
(C) STRANDEDNESS: single
(D) TOPOLOGY: unknown

(ii) MOLECULE TYPE: other nucleic acid
(A) DESCRIPTION: /desc = "primer"

(iii) HYPOTHETICAL: NO

(iv) ANTI-SENSE: NO

(vi) ORIGINAL SOURCE:
(A) ORGANISM: primer

(xi) SEQUENCE DESCRIPTION: SEQ ID NO: 23:

ACCACCAACG GTGTTCTTGC TGTTGA 26

(2) INFORMATION FOR SEQ ID NO: 24:

(i) SEQUENCE CHARACTERISTICS:
(A) LENGTH: 27 base pairs
(B) TYPE: nucleic acid
(C) STRANDEDNESS: single
(D) TOPOLOGY: unknown

(ii) MOLECULE TYPE: other nucleic acid
(A) DESCRIPTION: /desc = "primer"

(iii) HYPOTHETICAL: NO

(iv) ANTI-SENSE: NO

(vi) ORIGINAL SOURCE:
(A) ORGANISM: primer

(xi) SEQUENCE DESCRIPTION: SEQ ID NO: 24:

GCATTACATG TTAATTATTA CATGCTT 27

(2) INFORMATION FOR SEQ ID NO: 25:

(i) SEQUENCE CHARACTERISTICS:
(A) LENGTH: 28 base pairs
(B) TYPE: nucleic acid
(C) STRANDEDNESS: single
(D) TOPOLOGY: unknown

(ii) MOLECULE TYPE: other nucleic acid
(A) DESCRIPTION: /desc = "primer"

(iii) HYPOTHETICAL: NO

(iv) ANTI-SENSE: NO

(vi) ORIGINAL SOURCE:
(A) ORGANISM: primer

(xi) SEQUENCE DESCRIPTION: SEQ ID NO: 25:

GTGATACGAG TTTCACCGCT AGCGAGAC 28

(2) INFORMATION FOR SEQ ID NO: 26:

(i) SEQUENCE CHARACTERISTICS:
(A) LENGTH: 24 base pairs
(B) TYPE: nucleic acid
(C) STRANDEDNESS: single
(D) TOPOLOGY: unknown

(ii) MOLECULE TYPE: other nucleic acid
(A) DESCRIPTION: /desc = "primer"

(iii) HYPOTHETICAL: NO

(iv) ANTI-SENSE: NO

(vi) ORIGINAL SOURCE:
(A) ORGANISM: primer

(xi) SEQUENCE DESCRIPTION: SEQ ID NO: 26:

TACCTTGCGT GGACCAAAGA CTCC 24

(2) INFORMATION FOR SEQ ID NO: 27:

(i) SEQUENCE CHARACTERISTICS:
(A) LENGTH: 24 base pairs
(B) TYPE: nucleic acid
(C) STRANDEDNESS: single
(D) TOPOLOGY: unknown

(ii) MOLECULE TYPE: other nucleic acid
(A) DESCRIPTION: /desc = "primer"

(iii) HYPOTHETICAL: NO

(iv) ANTI-SENSE: NO

(vi) ORIGINAL SOURCE:
(A) ORGANISM: primer

(xi) SEQUENCE DESCRIPTION: SEQ ID NO: 27:

ATGGCTTCCG CTCAAGTGAA GTCC 24

(2) INFORMATION FOR SEQ ID NO: 28:

(i) SEQUENCE CHARACTERISTICS:
(A) LENGTH: 24 base pairs
(B) TYPE: nucleic acid
(C) STRANDEDNESS: single
(D) TOPOLOGY: unknown

(ii) MOLECULE TYPE: other nucleic acid
(A) DESCRIPTION: /desc = "primer"

(iii) HYPOTHETICAL: NO

(iv) ANTI-SENSE: NO

(vi) ORIGINAL SOURCE:
(A) ORGANISM: primer

(xi) SEQUENCE DESCRIPTION: SEQ ID NO: 28:

CGAGACCCAT AACGAGGAAG CTCA 24

(2) INFORMATION FOR SEQ ID NO: 29:

(i) SEQUENCE CHARACTERISTICS:
(A) LENGTH: 24 base pairs
(B) TYPE: nucleic acid
(C) STRANDEDNESS: single
(D) TOPOLOGY: unknown

(ii) MOLECULE TYPE: other nucleic acid
(A) DESCRIPTION: /desc = "primer"

(iii) HYPOTHETICAL: NO

(iv) ANTI-SENSE: NO

(vi) ORIGINAL SOURCE:
(A) ORGANISM: primer

(xi) SEQUENCE DESCRIPTION: SEQ ID NO: 29:

ATTGCGTGAT TTCGATCCTA ACTT 24

(2) INFORMATION FOR SEQ ID NO: 30:

(i) SEQUENCE CHARACTERISTICS:
(A) LENGTH: 24 base pairs
(B) TYPE: nucleic acid
(C) STRANDEDNESS: single
(D) TOPOLOGY: unknown

(ii) MOLECULE TYPE: other nucleic acid
(A) DESCRIPTION: /desc = "primer"

(iii) HYPOTHETICAL: NO

(iv) ANTI-SENSE: NO

(vi) ORIGINAL SOURCE:
(A) ORGANISM: primer

(xi) SEQUENCE DESCRIPTION: SEQ ID NO: 30:

GAGAGATGTC GATAGAGGTC TTCT 24

(2) INFORMATION FOR SEQ ID NO: 31:

(i) SEQUENCE CHARACTERISTICS:
(A) LENGTH: 24 base pairs
(B) TYPE: nucleic acid
(C) STRANDEDNESS: single
(D) TOPOLOGY: unknown

(ii) MOLECULE TYPE: other nucleic acid
(A) DESCRIPTION: /desc = "primer"

(iii) HYPOTHETICAL: NO

(iv) ANTI-SENSE: NO

(vi) ORIGINAL SOURCE:
(A) ORGANISM: primer

(xi) SEQUENCE DESCRIPTION: SEQ ID NO: 31

GGTGGAGCAC GACACACTTG TCTA 24

(2) INFORMATION FOR SEQ ID NO: 32:

(i) SEQUENCE CHARACTERISTICS:
(A) LENGTH:18 base pairs
(B) TYPE: nucleic acid
(C) STRANDEDNESS: single
(D) TOPOLOGY: unknown

(ii) MOLECULE TYPE: other nucleic acid
(A) DESCRIPTION: /desc = "primer"

(iii) HYPOTHETICAL: NO

(iv) ANTI-SENSE: NO

(vi) ORIGINAL SOURCE:
(A) ORGANISM: primer

(xi) SEQUENCE DESCRIPTION: SEQ ID NO: 32:

GTCTCAATGT AATGGTTA 18

## Description

The present invention relates to recombinant DNA technology, and in particular to the production of transgenic plants which exhibit substantial resistance or substantial tolerance to herbicides when compared with non transgenic like plants.

Plants which are substantially "tolerant" to a herbicide when they are subjected to it provide a dose/response curve which is shifted to the right when compared with that provided by similarly subjected non tolerant like plants. Such dose/response curves have "dose" plotted on the x-axis and "percentage kill", "herbicidal effect" etc. plotted on the y-axis. Tolerant plants will require more herbicide than non tolerant like plants in order to produce a given herbicidal effect. Plants which are substantially "resistant" to the herbicide exhibit few, if any, necrotic, lytic, chlorotic or other lesions when subjected to the herbicide at concentrations and rates which are typically employed by the agrochemical community to kill weeds in the field. Plants which are resistant to a herbicide are also tolerant of the herbicide. The terms "resistant" and "tolerant" are to be construed as "tolerant and/or resistant" within the context of the present application.

According to the present invention there is provided a polynucleotide comprising at least a first region encoding a first protein capable of conferring on a plant, or tissue comprising it, resistance or tolerance to a first herbicide, and a second region encoding a second protein likewise capable of conferring resistance to a second herbicide, with the *provisos* (i) that the polynucleotide does not encode a fusion protein comprising only a 5-enol-pyruvyl-3-phosphoshikimate synthetase (EPSPS) and a glutathione S transferase (GST); (ii) that the polynucleotide does not comprise only regions encoding superoxide dismutase (SOD) and glutathione S transferase (GST); and (iii) that the polynucleotide does not comprise only regions encoding GST and phosphinothricin acetyl transferase (PAT).

In a preferred embodiment of the invention the regions comprised by the polynucleotide are each under expression control of a plant operable promoter and terminator. Such promoters and terminators are well known to the skilled man who will choose them according to his particular needs. For example, suitable promoters include the 35S CaMV or FMV promoters, and the arabidopsis and maize ubiquitin promoters. Preferably, the promoters are constitutive. This avoids any need for external induction and means that the plant is permanently tolerant of or resistant to each corresponding herbicide. DNA encoding the herbicide resistance genes may also be included in a plant transformation vector under the control of an inducible promoter, to give inducible herbicide resistance in the transgenic plants. Such promoters include the chemically-inducible known GST-27 promoter by which resistance may be switched on by application of a suitable inducer (such as a chemical safener). In certain circumstances, the ability to express or to increase herbicide resistance only when required may be advantageous. For example, during rotation of crops, individuals of the first crop species may grow the following year in the field to be cultivated with a second crop species. A herbicide may be used to destroy these un-induced and still susceptible "volunteer" plants. Induction of herbicide resistance gene expression only when herbicide resistance is required (that is, just before application of a herbicide) may also be metabolically more efficient in some circumstances as the plant then produces resistance polypeptides only when required. Suitable inducible promoters further include the tetracycline-inducible promoter, the lac bacterial repressor/operator system, the glucocorticoid receptor, together with dexamethasone, copper and salicylic acid-inducible promoters, promoters based on the ecdysone receptor, as described in International Patent Application No. PCT/GB96/01195, and the so-called A1c promoter, as described in International Patent Publication No. WO93/21334.

In a particularly preferred embodiment of the invention, at least one of the regions comprised by the polypeptide provides for resistance to a pre-emergence herbicide and at least one of the regions provides for resistance to a post emergence herbicide. Whilst the skilled man does not need a definition of pre-emergence and post emergence, by "pre-emergence" is meant applied before the germinating seed emerges above the soil surface, ie before any plant material is visible above the ground. Post emergence means applied after the seedling is visible above the surface of the soil.

The pre-emergence herbicide may be selected from the group consisting of a dinitroaniline herbicide, bromacil, flupoxam, picloram, fluorochloridone, tetrazolinones including N-carbamoyltetrazolinones such as those described in EP-A-612,735, sulcatrione, norflurazone, RP201772, atrazine or another triazine, iminothiadozole, diflufenicon, sulfonyl urea, imidazolinone, thiocarbamate, triazine, uracil, urea, triketone, isoxazole, acetanilide, oxadiazole, the phosphosulfonate herbicides described in EP-A-511,826, triazinone, sulfonanilide, amide, oxyacetamides such as fluthiamide, anilide and triazolinone type herbicide. Examples of triketone herbicides include 2-(2-Nitro-4-trifluoromethylbenzoyl)-cyclohexane-1,3-dione
2-(2-Chloro-4-methanesulphonylbenzoyl)-cyclohexane-1, 3-dione,
2-(2-2 Nitro-4-methanesulphonylbenzoyl)-cyclohexane-1,3-dione,
[ 5-cyclopropyl-4-(2-methylsulphonyl-4-trifluoromethylbenzoyl)isoxazole, etc.

For the avoidance of doubt, by "triketone herbicide" is meant any compound capable of inhibiting a 4-hydroxyphenyl pyruvate (or pyruvic acid) dioxygenase (HPPD). Within the context of the present invention the terms 4-hydroxy phenyl pyruvate (or pyruvic acid) dioxygenase (4-HPPD) and p-hydroxy phenyl pyruvate (or pyruvic acid) dioxygenase (p-OHPP) are synonymous.

The post-emergence herbicide may be selected from the group consisting of glyphosate and salts thereof, glufosinate, diphenyl ether, asulam, bentazon, bialaphos, bromacil, sethoxydim or another cyclohexanedione, dicamba, fosamine, flupoxam, phenoxy propionate, quizalofop or another aryloxy-phenoxypropanoate, picloram, fluormetron, atrazine or another triazine, metribuzin, chlorimuron, chlorsulfuron, flumetsulam, halosulfuron, sulfometron, imazaquin, imazethapyr, isoxaben, imazamox, metosulam, pyrithrobac, rimsulfuron, bensulfuron, nicosulfuron, fomesafen, fluroglycofen, KIH9201, ET751, carfentrazone, ZA1296, ICIA0051, RP201772, flurochloridone, norflurazon, paraquat, diquat, bromoxynil and fenoxaprop. Particularly preferred combinations of these herbicides to which the polynucleotide of the invention is capable of conferring resistance (or to which the plants of the invention are resistant or tolerant) are: (i) glyphosate and diphenyl ether or acetanalide type herbicides: (ii) glyphosate and/or glufosinate and anilide and/or triazolinone type herbicides; (iii) triketones and glyphosate and/or glufosinate; (iv) glyphosate and/or glufosinate and triketones and anilide type herbicides; (v) glyphosate and/or glufosinate and a PDS inhibitor (such as the compounds of formulas I-III depicted below).

The proteins encoded by the said regions of the polynucleotide may be selected from the group consisting of glyphosate oxido-reductase (GOX), 5-enol-pyruvyl-3-phosphoshikimate synthetase (EPSPS), phosphinothricin acetyl transferase (PAT), hydroxyphenyl pyruvate dioxygenase (HPPD), glutathione S transferase (GST), cytochrome P450, Acetyl-COA carboxylase (ACC), Acetolactate synthase (ALS), protoporphyrinogen oxidase (protox), dihydropteroate synthase, polyamine transport proteins, superoxide dismutase (SOD), bromoxynil nitrilase (BNX), phytoene desaturase (PDS), the product of the *tƒdA* gene obtainable from *Alcaligenes eutrophus,* and mutagenised or otherwise modified variants of the said proteins. The product of the said *tƒdA* gene is a dioxygenase which is capable of oxidising phenoxycarboxylic acids, such as 2,4-D to the corresponding phenol. The EPSPS enzyme may be a so called class II EPSPS, as described in European Patent No. 546,090. Alternatively, and/or additionally, it may be mutated so as to comprise amino acid substitutions at certain positions which are known to result in enhanced resistance to glyphosate (and agriculturally acceptable salts thereof). For example, the EPSPS may have at least the residues Thr, Pro, Gly and Ala at positions corresponding to 174, 178, 173 and 264 with respect to the EPSPS depicted in SEQ ID No. 9 alerted as follows:
(i) Thr 174 - Ile
(ii) Pro 178 - Ser
(iii) Gly 173 - Ala
(iv) Ala 264 - Thr
wherein (i) Thr 174 occurs within a sequence comprising contiguously Ala -Gly-Thr-Ala-Met; (ii) Pro 178 occurs within a sequence comprising contiguously Met-Arg-Pro-Leu-Thr; (iii) Gly 173 occurs within a sequence comprising contiguously Asn-Ala-Gly-Thr-Ala; and (iv) Ala 264 occurs within a sequence comprising contiguously Pro-Leu-Ala-Leu-Gly. Additionally, the terminal Gly residue within the sequence motif Glu-Arg-Pro-AA1-AA2-Leu-Val-AA3-AAA4-Leu-AA5-AA6-AA7-Gly- in a region of the EPSPS enzyme corresponding to that spanning positions 192 to 232 in SEQ ID No. 9 may be replaced by either an Asp or Asn residue.

In one embodiment of the polynucleotide, the region encoding the HPPD enzyme has the sequence depicted in SEQ ID Nos. 1 or 3, or alternatively is complementary to one which when incubated at a temperature of between 60 and 65°C in 0.3 strength citrate buffered saline containing 0.1% SDS followed by rinsing at the same temperature with 0.3 strength citrate buffered saline containing 0.1% SDS still hybridises with the sequence depicted in SEQ ID No. 1 or 3 respectively.

When the test and inventive sequences are double stranded the nucleic acid constituting the test sequence preferably has a TM within 15°C of that of the said SEQ ID No. 1 sequence. In the case that the test and SEQ ID No. 1 sequences (or test and SEQ ID No. 3 sequences) are mixed together and are denatured simultaneously, the TM values of the sequences are preferably within 5°C of each other. More preferably the hybridisation is performed under relatively stringent conditions, with either the test or inventive sequences preferably being supported. Thus either a denatured test or inventive sequence is preferably first bound to a support and hybridisation is effected for a specified period of time at a temperature of between 60 and 65°C in 0.3 strength citrate buffered saline containing 0.1% SDS followed by rinsing of the support at the same temperature but with 0.1 strength citrate buffered saline. Where the hybridisation involves a fragment of the inventive sequence, the hybridisation conditions may be less stringent, as will be obvious to the skilled man.

When the polynucleotide comprises an HPPD gene capable of conferring resistance to triketone herbicides, plant material transformed therewith may be subjected to a triketone herbicide and visually selected on the basis of a colour difference between the transformed and non transformed material when subjected to the said herbicide. Thus the non-transformed material may become and stay white when subjected to the selection procedure, whereas the transformed material may become white but later turn green, or may remain green, likewise, when subjected to the said selection procedure.

A further embodiment of the polynucleotide of the invention includes a further region encoding a protein capable of providing the plant with resistance or tolerance to insects, desiccation and/or fungal, bacterial or viral infections. The proteins encoded by such regions are known to the skilled man and include the delta endotoxin from *Bacillus thuringiensis* and the coat proteins from viruses, for example.

The polynucleotide may comprise sequences 5' of and contiguous with the said regions, which sequences encode (i) a peptide which is capable of targeting the translation products of the regions to plastids such as chloroplasts, mitochondria, other organelles or plant cell walls; and/or (ii) non-translated translational enhancing sequences. Suitable targeting sequences encode chloroplast transit peptides, particularly in the case that the herbicide resistance conferring region immediately down-stream of it is an EPSPS or GOX enzyme. Translational expression of the protein encoding sequences contained within the polynucleotide may be relatively enhanced by including known non translatable translational enhancing sequences 5' of the said protein encoding regions. The skilled man is very familiar with such enhancing sequences, which include the TMV-derived sequences known as omega, and omega prime, as well as other sequences derivable, *inter alia*, from the regions 5' of other viral coat protein encoding sequences, such as that of the Tobacco Etch virus. It may be desirable, having regard to the expression of nucleotide sequences *in planta,* to modify the sequences encoding known proteins capable of conferring resistance to herbicides. Accordingly the invention also includes a polynucleotide as indicated above, but which is modified in that mRNA instability motifs and/or fortuitous splice regions are removed, or plant preferred codons are used so that expression of the thus modified polynucleotide in a plant yields substantially similar protein having a substantially similar activity/function to that obtained by expression of the unmodified polynucleotide in the organism in which the protein encoding regions of the unmodified polynucleotide are endogenous, with the *proviso* that if the thus modified polynucleotide comprises plant preferred codons, the degree of identity between the protein encoding regions within the modified polynucleotide and like protein encoding regions endogenously contained within the said plant and encoding substantially the same protein is less than about 70%.

The invention further includes a vector comprising the said polynucleotide.

The invention still further provides plants which comprise at least two nucleotide sequences encoding proteins capable of conferring resistance to at least two herbicides and which have been regenerated from material which has been transformed with the polynucleotide or vector of the invention. Transformation techniques are well known and include particle mediated biolistic transformation, Agrobacterium-mediated transformation, protoplast transformation (optionally in the presence of polyethylene glycols); sonication of plant tissues, cells or protoplasts in a medium comprising the polynucleotide; micro-insertion of the polynucleotide into totipotent plant material (optionally employing the known silicon carbide "whiskers" technique), electroporation and the like. The transformed inventive plants include small grain cereals, oil seed crops, fibre plants, fruit, vegetables, plantation crops and trees. Particularly preferred such plants include soybean, cotton, tobacco, sugarbeet, oilseed rape, canola, flax, sunflower, potato, tomato, alfalfa, lettuce, maize, wheat, sorghum, rye, bananas, barley, oat, turf grass, forage grass, sugar cane, pea, field bean, rice, pine, poplar, apple, grape, citrus or nut plants and the progeny, seeds and parts of such plants.

The invention still further provides plant material which comprises nucleic acid sequences comprising regions encoding at least two proteins capable of conferring upon the material resistance to at least two herbicides, with the *provisos* that the material that the material does not contain a polynucleotide which encodes a fusion protein comprising only a 5-enol-pyruvyl-3-phosphoshikimate synthetase (EPSPS) and a glutathione S transferase (GST); (ii) that the material does not contain a polynucleotide which comprises only regions encoding superoxide dismutase (SOD) and glutathione S transferase (GST); (iii) that the material does not contain a polynucleotide which comprises only regions encoding GST and phosphinothricin acetyl transferase (PAT); and (iv), that when the plant from which the material is derived is sugar beet, the herbicide resistance or tolerance conferring genes which it comprises are not solely EPSPS and PAT.

The material may be regenerated into morphologically normal fertile whole plants, by means known to the skilled man. In a preferred embodiment of the material, at least one of the regions encodes a protein capable of conferring resistance to a pre-emergence type herbicide, and at least one of the regions encodes a protein capable of providing resistance to a post emergence type herbicide. Such protein encoding regions and herbicides have been discussed above. The skilled man will recognise that multiple herbicide resistance conferring regions may be present in plants (or parts thereof) as a consequence of the crossing of a first plant comprising a polynucleotide encoding a first protein capable of conferring resistance to a first herbicide with a second plant which comprises a polynucleotide encoding a second protein capable of conferring resistance to a second herbicide (see the experimental part of the application). Preferred combinations of herbicide resistance conferring genes are (i) an HPPD gene and an EPSPS or GOX gene; (ii) an HPPD gene and a PAT gene; (iii) a GST gene and an EPSPS/GOX gene; (iv) an EPSPS/GOX gene and a PAT gene; (iv) an HPPD gene, a GOX and/or EPSPS gene, and a PAT gene; (v) an ACC'ase gene and a PAT and/or EPSPS gene; (vi) a PDS gene and a PAT and/or EPSPS and/or GOX gene; (vii)), the *tƒd*A gene obtainable from *Alcaligenes eutrophus* and an EPSPS and/or GOX and/or PAT and/or PDS gene. In addition each of these combinations may have one or more of the herbicide genes replaced by a SOD, protox and/or ALS gene. Such plants are referred to in this application as plants of the invention.

The invention also includes a method of selectively controlling weeds in a field comprising weeds and crop plants, wherein the crop plants comprise (i) a polynucleotide comprising at least a first region encoding a first protein capable of conferring on a plant, or tissue comprising it, resistance or tolerance to a first herbicide, and a second region encoding a second protein likewise capable of conferring resistance to a second herbicide, with the *provisos* (i) that the polynucleotide does not encode a fusion protein comprising only a 5-enol-pyruvyl-3-phosphoshikimate synthetase (EPSPS) and a glutathione S transferase (GST); (ii) that the polynucleotide does not comprise only regions encoding superoxide dismutase (SOD) and glutathione S transferase (GST); (iii) that the polynucleotide does not comprise only regions encoding GST and phosphinothricin acetyl transferase (PAT); and (iv), that when the crop plant is sugar beet, the herbicide resistance or tolerance conferring genes which it comprises are not solely EPSPS and PAT; or (ii) a polynucleotide comprising at least a first region encoding a first protein capable of conferring on a plant, or tissue comprising it, resistance or tolerance to a first herbicide, and a polynucleotide comprising a second region encoding a second protein likewise capable of conferring resistance to a second herbicide, with the *provisos* (i) that the polynucleotide does not encode a fusion protein comprising only a 5-enol-pyruvyl-3-phosphoshikimate synthetase (EPSPS) and a glutathione S transferase (GST); (ii) that the polynucleotide does not comprise only regions encoding superoxide dismutase (SOD) and glutathione S transferase (GST); (iii) that the polynucleotide does not comprise only regions encoding GST and phosphinothricin acetyl transferase (PAT); and (iv), that when the crop plant is sugar beet, the herbicide resistance or tolerance conferring genes which it comprises are not solely EPSPS and PAT, the method comprising application to the field of at least one of the said herbicides in an amount sufficient to control the weeds without substantially affecting the crop plants. The herbicide resistance conferring genes may be present on separate polynucleotides within the plant. In a preferred method the plant contains genes encoding an EPSPS and/or GOX enzyme and an HPPD enzyme, the method comprising application to the field of glyphosate and a triketone herbicide in an amount sufficient to control the weeds without substantially affecting the crop plants. In a further embodiment of the method, the plant contains genes encoding an EPSPS and/or GOX enzyme and a phosphinothricin acetyl transferase, the method comprising application to the field of glyphosate and glufosinate. In a further embodiment of the method, the plant contains genes encoding an EPSPS and/or GOX enzyme and a phosphinothricin acetyl transferase and an HPPD enzyme, the method comprising application to the field of glyphosate and glufosinate and a triketone herbicide. In a further embodiment of the method, the plant contains genes encoding an EPSPS and/or GOX enzyme and/or a phosphinothricin acetyl transferase and a glutathione S transferase, the method comprising application to the field of glyphosate and/or glufosinate and an anilide herbicide such as acetochlor, for example. In a further embodiment of the method, the plant contains genes encoding an ACC'ase and a PAT and/or EPSPS enzyme, the method comprising application to the field of a fluazifop type herbicide and glufosinate and/or glyphosate. In a still further embodiment of the method, the plant contains genes encoding the product of the *tƒdA* gene (optionally codon optimised) obtainable from *Alcaligenes eutrophus* and an EPSPS and/or GOX and/or PAT and/or PDS enzyme, the method comprising application to the field of 2,4 D and glyphosate and/or glufosinate and/or a herbicidal inhibitor of phytoene desaturase. In addition each of these combinations may have one or more of the herbicide genes replaced by a SOD, protox and/or ALS gene.

In a particularly preferred embodiment of this inventive method, a pesticidally effective amount of one or more of an insecticide, fungicide, bacteriocide, nematicide and anti-viral is applied to the field either prior to or after application to the field of one or more herbicides.

The present invention further provides a method of producing plants which are substantially tolerant or substantially resistant to two or more herbicides, comprising the steps of:
(i) transforming plant material with the polynucleotide or vector of the invention;
(ii) selecting the thus transformed material; and
(iii) regenerating the thus selected material into morphologically normal fertile whole plants.

The plants of the invention may optionally be obtained by a process which involves transformation of a first plant material with a first herbicide resistance conferring sequence, and transformation of a second plant material with a second herbicide resistance conferring sequence, regeneration of the thus transformed material into fertile whole plants and cross pollination of the plants to result in progeny which comprises both the said first and second herbicide resistance genes. Optionally the first and/or second material may have been prior transformed with polynucleotides comprising regions encoding one or more of a herbicide resistance conferring protein, an insecticidal protein, an anti-fungal protein, an anti-viral protein, and/or a protein capable of conferring upon a plant improved desiccation tolerance.

The invention still further provides the use of the polynucleotide or vector of the invention in the production of plant tissues and/or morphologically normal fertile whole plants (i) which are substantially tolerant or substantially resistant to two or more herbicides.

The invention still further provides the use of the polynucleotide or vector of the invention in the production of a herbicidal target for the high throughput *in vitro* screening of potential herbicides. The protein encoding regions of the polynucleotide may be heterologously expressed in *E. coli* or yeast.

The invention still further includes plant tissue transformed with a polynucleotide comprising the sequence depicted in SEQ ID No. 1 and encoding a dioxygenase. This may be the only herbicide resistance conferring gene within the material. The material may be regenerated into morphologically normal fertile plants using known means. In a particularly preferred embodiment of the transformed tissue, the polynucleotide which encodes a protein having a substantially similar activity to that encoded by SEQ ID No. 1, is complementary to one which when incubated at a temperature of between 60 and 65°C in 0.3 strength citrate buffered saline containing 0.1% SDS followed by rinsing at the same temperature with 0.3 strength citrate buffered saline containing 0.1% SDS still hybridises with the sequence depicted in SEQ ID No. 1.

The invention will be further apparent from the following description taken in conjunction with the associated figures and sequence listings.
SEQ ID No. 1 shows a DNA sequence, isolated from *Synechocystis sp,* which encodes *a*n enzyme (depicted as SEQ ID No. 2) having the activity of a p-hydroxyphenyl pyruvic acid dioxygenase.
SEQ ID No. 3 shows a DNA sequence, isolated from *Pseudomonas* spp. 87/79, in which nucleotides 1217 to 2290 encode an enzyme (depicted as SEQ ID No. 4) having the activity of a p-hydroxyphenyl pyruvic acid dioxygenase.
SEQ ID Nos. 5 and 6 depict one form of the minimally redundant synthetic PCR primers (see reference to HPPD-P4 and HPPD-REV1 below) which were used to isolate SEQ ID No 3 from the bacterial genome.
SEQ ID Nos. 7 and 8 are also synthetic PCR primers which were used to modify the SEQ ID No. 3 sequence so that it could be incorporated into the desired plant transformation vector.
SEQ ID No. 9 shows the amino acid sequence of an EPSPS enzyme (including chloroplast signal peptide) from petunia.
SEQ ID Nos. 10-32 are PCR primers or poly-linkers which are inserted into restricted plasmids to enable the production of constructs comprising multiple genes capable of conferring resistance to herbicides.

Figure 1 shows a schematic diagram of the clone comprising the sequence depicted in SEQ ID No. 3, in which three open reading frames are identified: the first starting at nucleotide 15 and ending at nucleotide 968; the second starting at nucleotide 215 and ending at nucleotide 1066 and the third starting at nucleotide 1217 and ending at nucleotide 2290 in SEQ ID No. 3. The Figure also shows the restriction sites contained within the sequence which are engineered by use of the primers designated as SEQ ID Nos. 7 and 8. Figure 2 schematically depicts the production of a 4-HPPD containing plant expression cassette in which the PCR edited DNA fragment of Figure 1 is restricted with the enzymes *Nco*1 and *Kpn*1, then ligated into a vector (pMJB1) also restricted with *Nco*1 and *Kpn*1. Figure 3 is a schematic representation showing how the plant transformation binary vector pBin 19 is engineered to contain the 4-HPPD expression cassette of Figure 2.
Figure 4 shows a schematic diagram of the clone comprising the sequence depicted in SEQ ID No. 1. Figure 5 schematically depicts the production of a 4-HPPD containing plant expression cassette in which a PCR edited DNA fragment of Figure 4 is restricted with the enzymes *Nco* 1 and *Kpn*1, then ligated into a vector (pMJB1) also restricted with *Nco* 1 and *Kpn*1.
Figure 6 shows schematically the construction of a plasmid vector, used in *Agrobacterium* transformation and also includes maps of plasmids pJR1Ri and pGST-27Bin;
Figure 7 shows GST activity in transformed tobacco subjected to four herbicides
Figure 8 is a graph comparing damage to wild type plants and a GST-27 line following metolachlor treatment at 1400 g/ha for 3 weeks;
Figure 9 is a map of the plasmid pDV3-puc;
Figure 10 is a map of the plasmid pDV6-Bin;
Figure 11 is a map of plasmid pUB-1 containing the Ubiquitin promoter fragment PCRed from maize, a 2Kb fragment is cloned into pUC 19 and the junctions are sequenced to confirm the presence of the Ubiquitin promoter;
Figure 12 is a map of plasmid pIE98;
Figure 13 is a map of plasmid pIGPAT;
Figure 14 is a map of plasmid pCAT10;
Figure 15 is a map of plasmid pCAT11;
Figure 16 is a map of plasmid pPG6;
Figure 17 depicts part of the pMV1 plasmid.

### EXAMPLE 1

### Cloning of the 4-HPPD gene from Pseudomonas spp, transformation of the gene into plant material and the production of triketone herbicide resistant plants.

The amino acid sequence of 4-HPPD purified from *Pseudomonas fluorescens* PJ-874, grown on tyrosine as the sole carbon source is known. (Ruetschi *et al*., Eur. J. Biochem 1992 202(2):459-466): Using this sequence minimally redundant PCR primers are designed with which to amplify a large but incomplete segment of the 4-HPPD gene from genomic DNA from a different bacterial strain *(Pseudomonas fluorescens* strain 87-79). The skilled man recognises what is meant by the term "minimally redundant primers", the redundancy being represented by squared brackets in the sequences depicted below. One example of each of the respective primers (corresponding to a 5' and 3' location within the HPPD gene) is given in each of SEQ ID Nos. 3 and 4.

Primer 1 (SEQ ID No. 5) which is a 17mer is designed from a knowledge of the sequence of amino acids 4-9 of the published protein sequence (see above) and Primer 2 (SEQ ID No. 6), likewise a 17mer, is designed from a knowledge of residues 334 to 339.

Primer 1 (HPPD-P4) has the sequence 5'TA[T/C] GA[G/A] AA[T/C] CC[T/C/G/A] ATG GG and primer 2 (HPPD-REV1) has the sequence 5'GC[T/C] TT[G/A] AA[G/A] TT[T/C/G/A] CC[T/C] TC. 100 ng genomic of DNA from Pseudomonas 87-79 was prepared using standard protocols and mixed with 100 pmol of each primer. The mixture is PCR amplified (35 cycles) using a Taq polymerase and other standard reagents under the following DNA synthesis and dissociation conditions:
94°C x 1.5 min
55°C x 2 min
74°C x 3 min

The amplified fragment comprises a region containing 3 codons from the 5' end, and about 30 codons from the 3' end of the coding region of the 4HPPD gene. The PCR product is blunt end cloned in the housekeeping vector pGEM3Z-f(+) using standard procedures.

Partial sequencing confirms that the cloned PCR fragment is 4-HPPD specific. The derived amino sequence contains several discrepancies compared with sequence published in respect of the *Pseudomonas fluorescens* PJ-874 enzyme. This partial fragment of the 4-HPPD gene gives negative hybridisation signals in genomic Southern blots on plant DNA under low stringency hybridisation/wash conditions. A 900 bp *Eco*R1 / *Eco*R1 fragment is excised from the centre of the previously cloned partial gene to use as a probe. Southern blots, using a variety of enzymes to restrict the genomic DNA, are hybridised with the radiolabelled fragment.

*Bcl*1 restricted DNA gives a single positive band of approx. 2.5 kb which is sufficient to contain the entire gene plus flanking regions of untranslated DNA. Genomic DNA is restricted with *Bcl*1 and electrophoresed on a preparative agarose gel. The region of digested DNA containing fragments in the size range 2 - 3 Kb is cut out and the DNA electro-eluted. The recovered DNA is cloned into the *Bam*H1 (which is compatible with *Bcl* 1) site of pUC18. Colony blots are probed with the 900 bp fragment and 12 positives are isolated. Minipreps are made from these, and cut with *Eco*R1 to look for the diagnostic 900 bp band. Of 12 colonies, 7 formed a brown pigment when grown overnight in LB to make the minipreps, 5 of these are positive for the 900 bp band, the other 5 minipreps are negative and do not produce the brown pigment. The formation of the "brown pigment" is associated with the heterologous expression of a 4-HPPD gene.

Restriction analysis shows that the cloned insert was 2.5 kb long with about 1.2 kb DNA upstream of the 4-HPPD gene and 400 bp downstream. The ends of the gene are sequenced using appropriate primers and primers from pUC18. Such sequencing proves the gene to be intact and present in both orientations with regard to the pUC 18 polylinker site.

SDS-PAGE on bacterial cell lysates shows that a new protein is present with a size of 40 kDa, which is correct for a 4-HPPD. A large band is present in extracts from cells having the gene inserted in a first orientation such that the gene is expressed from the plac promoter in the vector. No 40 kD band is obviously visible when the lysate is obtained from the cells in which the gene is in the opposite orientation, although both clones produced the brown pigment suggesting the presence of the active protein in both cell types. The 40 kDa recombinant protein is present in the soluble rather than the insoluble protein fraction. The clone in which the gene is in the second orientation is subjected to automated DNA sequence analysis to reveal the sequence depicted in SEQ ID No. 3. This sequence is edited to introduce several unique restriction sites to facilitate its assembly into a vector suitable for plant transformation work. The editing oligonucleotides, which are depicted in SEQ ID Nos. 7 and 8, are primer 3 (HPPDSYN1) 5'-GTTAGGTACCAGTCTAGACTGACCATGGCCGACCAATACGAAAACC-3'and primer 4 (HPPDSYN2) 5'TAGCGGTACCTGATCACCCGGGTTATTAGTCGGTGGTCAGTAC-3'.

### Expression of the Pseudomonas 4-HPPD gene in transgenic tobacco

The PCR edited DNA fragment is restricted with the enzymes *Nco*1 and *Kpn*1, then ligated into a vector (pMJB1) also restricted with *Nco*1 and *Kpn*1. pMJB1 is a pUC19 derived plasmid which contains the double CaMV35S promoter; a TMV omega enhancer and the NOS transcription terminator. A schematic representation of the resulting plasmid is shown in Figure 2. All of the DNA manipulations use standard protocols known to the man skilled in the art of plant molecular biology.

Bulk DNA is isolated and the 4-HPPD expression cassette (i.e. from the 2x35S to the nos 3' terminator), excised by partial restriction *Eco*R1 and then subjected to complete restriction with *Hind3.* This is to avoid cutting at an *Eco*R1 site within the 4-HPPD gene. Following preparative agarose gel electrophoresis, the required DNA fragment is recovered by electro-elution.

The 4-HPPD expression cassette is then ligated in to the binary vector pBin19 restricted with *Hind3* and *Eco*R1. The structure of the resulting plasmid is shown schematically in Figure 3.

DNA is isolated and used to transform *Agrobacterium tumefaciens* LBA4404 to kanamycin resistance again using standard procedures. Leaf discs/slices *of Nicotiana plumbaginifolia* var Samsun are subjected to *Agrobacterium-mediated* transformation using standard procedures. Transformed shoots are regenerated from kanamycin resistant callus. Shoots are rooted on MS agar containing kanamycin. Surviving rooted explants are re-rooted to provide about 80 kanamycin resistant transformed tobacco plants. The presence of the 4-HPPD gene (using pre-existing EDIT primers) is verified by PCR. About 60 plants are PCR positive.

Explants (i.e. a leaf plus short segment of stem containing the axillary bud) are placed into MS agar (+ 3% sucrose) containing various concentrations of ZA1206 (a triketone herbicide) from 0.02 to 2 ppm. Untransformed tobacco explants are fully bleached at 0.02 ppm. They do not recover following prolonged exposure to the herbicide. In these particular experiments, only the shoot which develops from the bud is bleached, the leaf on the explanted tissue remains green.

About 30 of the PCR+ve transformed plants tolerated 0.1 ppm of ZA1296 (about 5x the level which causes symptoms on wild-type tobacco) with no indication of bleaching. They root normally and are phenotypically indistinguishable from untransformed plants. A sub-set of the transformants was tolerant to 0.2 ppm and a few transformants tolerate concentrations of up to 0.5 - 1 ppm. Again these plants look normal and root well in the presence of herbicide. Some of the transformed plants can be initially bleached when subjected to the herbicide at the said higher concentrations, but on prolonged exposure they progressively "green up" and "recover".

A subset of the said herbicide resistant transgenic plants are treated with the known herbicide Isoxaflutole [5-cyclopropyl-4-(2-methylsulphonyl-4-trifluoromethylbenzoyl)isoxazole or RPA 210772]. Such plants are even more resistant to this herbicide than they are to that designated as ZA1296 thus clearly indicating that the plants are cross resistant to multiple classes of 4-HPPD inhibitor.

### EXAMPLE 2

### Cloning of the 4-HPPD gene from Synechocystis sp into plant material and regeneration of the material to yield triketone herbicide resistant plants.

The genome of *Synechocystis sp,* PCC6803 has been sequenced. In order to introduce unique restriction sites to facilitate its assembly into a vector suitable for plant transformation work 100 ng of genomic DNA from *Synechocystis sp.* is prepared using standard protocols and mixed with 100 pmol of two primers suitable for the PCR amplification (35 cycles) of the sequence specified in SEQ ID No. 1, using a thermostable DNA polymerase preferably with proof reading activity and other standard reagents under appropriate DNA synthesis and dissociation conditions, the following being typical:
94°C x 1.5 min
55°C x 2 min
74°C x 3 min

The amplified fragment comprises a region containing the coding region of the 4-HPPD gene. The PCR product is blunt end cloned in a standard housekeeping vector, such as, for example, pGEM3Z-f(+) using standard procedures.

Automated DNA sequence analysis confirms that the cloned PCR product is 4-HPPD specific. Some of the transformed colonies harbouring the cloned 4-HPPD gene form a brown pigment when grown overnight in LB. The formation of the "brown pigment" is associated with the heterologous expression of a 4-HPPD gene (Denoya *et al* 1994 J. Bacteriol. 176:5312-5319).

SDS-PAGE on bacterial cell lysates shows that they contain a new protein having the expected molecular weight for the 4-HPPD gene product. In a preferred embodiment the recombinant protein is either present in the soluble rather than the insoluble protein fraction, or else is manipulated to be so present. The clone is preferably subjected to automated DNA sequence analysis to confirm the absence of PCR derived artefacts.

### Heterologous expression of the Synechocystis sp. PCC6803 4-HPPD gene in E. coli

The PCR edited DNA fragment is restricted with suitable enzymes such as *Nco*1 and *Kpn*1, for example then ligated into an *E coli* expression vector (such as the known pET series) appropriately restricted. All of the DNA manipulations use standard protocols known to the man skilled in the art of molecular biology.

Suitable host strains such as BL21(DE3) or other DE3 lysogens harbouring the said vector express quantities of HPPD enzyme sufficient to provide for their use in high through put screening to identify alternative 4-HPPD inhibitors. HPPD purified from the said transformed host strain may be used in the provision of antisera for the analysis of plants transformed with a polynucleotide encoding 4-HPPD.

### Heterologous expression of the Synechocystis sp. PCC6803 4-HPPD gene in transgenic plants

The PCR edited DNA fragment is restricted with suitable enzymes such as *Nco*1 and *Kpn*1, for example then ligated into a suitable house keeping vector, such as pMJB1, to generate an expression cassette which contains an appropriate plant operable promoter and terminator. pMJB1 is a pUC19 derived plasmid which contains the double CaMV35S promoter; a TMV omega enhancer and the nos transcription terminator. A schematic representation of the resulting plasmid is shown in Figure 4.

The 4-HPPD expression cassette is then ligated in to the binary vector pBin19 restricted with *Hind3* and *Eco*R1. The structure of the resulting plasmid is shown schematically in Figure 5.

DNA is isolated and used to transform *Agrobacterium tumefaciens* LBA4404 to kanamycin resistance again using standard procedures. Potato and tomato tissue is subjected to *Agrobacterium-mediated* transformation using standard procedures. Transformed shoots are regenerated from kanamycin resistant callus. Shoots are rooted on MS agar containing kanamycin. Surviving rooted explants are re-rooted to provide about 80 kanamycin resistant transformed tobacco plants. The presence of the 4-HPPD gene (using pre-existing EDIT primers) is verified by PCR. A substantial number of PCR positive plants are selected for further analysis.

Explants (i.e. a leaf plus short segment of stem containing the axillary bud) are placed into MS agar (+ 3% sucrose) containing various concentrations of ZA1206 (a triketone herbicide) from 0.02 to 2 ppm. Untransformed explants are fully bleached at 0.02 ppm. They do not recover following prolonged exposure to the herbicide. In these particular experiments, only the shoot which develops from the bud is bleached, the leaf on the explanted tissue remains green.

About 30 of the PCR+ve transformed plants tolerated 0.1 ppm of ZA 1296 (about 5x the level which causes symptoms on wild-type tobacco) with no indication of bleaching. They root normally and are phenotypically indistinguishable from untransformed plants. A sub-set of the transformants is tolerant to 0.2 ppm and a few transformants tolerate concentrations of up to 0.5 - 1 ppm. Again these plants look normal and root well in the presence of herbicide. Some of the transformed plants can be initially bleached when subjected to the herbicide at the said higher concentrations, but on prolonged exposure they progressively "green up" and "recover".

A subset of the said herbicide resistant transgenic plants are treated with the known herbicide Isoxaflutole [5-cyclopropyl-4-(2-methylsulphonyl-4-trifluoromethylbenzoyl)isoxazole or RPA 210772]. Such plants are resistant to this herbicide and that designated as ZA1296 thus clearly indicating that the plants are cross resistant to multiple classes of 4-HPPD inhibitor.

### EXAMPLE 3

### Cloning of the GST gene into plant material and the generation of plants resistant to anilide and diphenyl ether type herbicides.

**Plants** Stocks of *Nicotiana tabacum* cv Samsum are kept on Musharige and Skoog medium (MS medium: MS salts (4.6 g/l) supplemented with 3% sucrose and 0.8% Bactoagar , pH 5.9). These plants, explants for the rooting assay and the seeds for the germination tests are grown in culture room at 25°C with 16 hours of light. When grown in the glasshouse, the plants are transferred into compost (John Innes compost number 3, Minster Brand products).
**Bacterial strains** *Escherichia coli,* strain DH5 (GIBCO BRL), is: F , 80dlacZ M15, (*lac*ZYAargF)U169, *deo*R, *rec*A1, *end*A1, *hsd*R17(r_{K}⁻ , m_{K}⁺), *supE44, thi-gyr*A96*rel*A1. *Agrobacterium tumefaciens,* strain LBA 4404, is used to transform tobacco leaves.
**Plasmids.** DNA of GST-27 is inserted in the 2.961 kb pBluescript® II SK (+/-) phagemid designated pIJ21-3A (Jepson *et al* 1994). pJR1Ri is a 12.6 kb plasmid. The pJR1Ri plasmid contains a bacterial kanamycin resistance marker (KAN). It possesses the 2 repetitive sequences of 25 bp: the right (RB) and the left (LB) borders. The T-DNA contains a kanamycin resistance marker gene driven by NOS promoter. The GST-27 protein encoding sequence is expressed under the control of the CaMV 35S promoter.
**Size markers.** A 1kb DNA ladder is used as a DNA size marker (Bethesda Research Laboratories Life Technologies, Inc) when digestions and PCR (polymerase chain reaction) products are checked on an agarose gel. The Rainbow protein molecular weight markers (Amersham) are loaded on polyacrylamide gels for the Western analyses, as is known to the skilled man.
**Chemicals**. The active ingredients acetochlor, alachlor and metolachlor are produced at ZENECA Agrochemicals (UK), Jealott's Hill Research Station. The technical ingredients are formulated in ethanol and used in the HPLC assay, the rooting assay and the germination test (see below).
**Plasmid construction.** The plasmid pIJ21-3A containing the DNA gene of GST-27 is digested by the restriction enzyme *Eco*RI (Pharmacia) in 1 x Tris acetate (TA) buffer. Digestions are checked on a 0.8% agarose gel. *EcoRI* digested fragments are ligated into the *Sma* 1 (Pharmacia) site of pJR1Ri (Figure 6) after filling the protruding ends with the Klenow DNA polymerase (Pharmacia). The calf-alkaline-phosphatase (C.A.P.) enzyme prevents the self-ligation of pJR1Ri before the ligation of the GST gene. Competent E. *coli* cells (DH5) are transformed with the plasmid by a heat shock method. They are grown on L-agar and kanamycin plates. Positive colonies are checked by PCR or by hybridization overnight at 42°C with labelled probes (α - ³²P dNTP). The melting temperature (Tm) of the probes is defined by adding 2°C for each A or T and 4°C for each G or C. The reaction is performed at the lowest Tm-5 C with the *Taq* polymerase (Ampli-Taq DNA polymerase, Perkin Elmer Cetus) according to the manufacturer's protocols. PCR conditions are set up for 35 cycles as following: denaturation of DNA at 94°C for 48 seconds, annealing at the lowest Tm for 1 minute and extension at 72°C for 2.5 minutes. Prior to the first cycle, the reaction starts at 85°C.
   Eight positive colonies are chosen and grown at 37°C on an overnight shaking L-broth and kanamycin culture. DNA from these cell culture is extracted and then purified from an ultracentrifugation at 50,000 rpm in a CsCl gradient.
   The orientation of the insert into pJR1Ri is checked by sequencing the region between the 35S promoter and the GST gene, according to the Sanger method, using the Sequenase® (version 2.0, United States Biochemical corporation) following the manufacturer's protocols. The resultant plasmid (pGST-27Bin) (Figure 6) is introduced into *Agrobacterium tumefaciens* strain LBA4404, using the freeze thaw method described by Hostlers et al 1987.
**Leaf transformation by *Agrobacterium*.** The transformation of pGST-27Bin into tobacco is performed according to the method described by Bevan 1984. 3-4 weeks old sterile culture of tobacco (*Nicotiana tabacum* cv Samsum), grown on MS, are used for the transformation. The leaves are incubated on NBM medium (MS medium supplemented with 1 mg/16-benzylamino purine (6-BAP), 0.1 mg/l naphthalene acetic acid (NAA)) and kanamycin for 1 day. This medium enables the growth of shoot from leaf. One day later, the edges of the leaves are cut off and leaves cut into pieces. They are then co-incubated with the transformed *Agrobacterium* cells, containing the pJR1RI plasmid with the insert (pGST-27Bin), suspension (strain LBA 4404) for 20 minutes. The pieces are returned to the plates containing the NBM medium afterwards. After 2 days, explants are transferred to culture pots containing the NBM medium supplemented with carbenicillin (500 mg/l) and kanamycin (100 mg/l). Five weeks later, 1 shoot per leaf disc is transferred on NBM medium supplemented with carbenicillin (200 mg/l) and kanamycin (100 mg/l). After 2-3 weeks, shoots with roots are transferred to fresh medium. 2 cuttings from each shoot are transferred to separate pots. One is kept as a tissue culture stock, the other one is transferred to soil for growth in the glasshouse after rooting. 42 independent transformants carrying the GST-27 construct are transferred to the glasshouse.
**Leaf DNA extraction for PCR reactions.** The presence of the transgene in the putative transformants is verified by PCR. Leaf samples are taken from 3-4 weeks old plants grown in sterile conditions. Leaf discs of about 5 mm in diameter are ground for 30 seconds in 200 µl of extraction buffer (0.5% sodium dodecyl sulfate (SDS), 250 mM NaCl, 100 mM Tris . HCI.(pH 8). The samples are centrifuged for 5 minutes at 13,000 rpm and afterwards 150 µl of isopropanol is added to the same volume of the top layer. The samples are left on ice for 10 minutes, centrifuged for 10 minutes at 13,000 rpm and left to dry. Then they are resuspended in 100µl of deionised water, 15µl of which is used for the PCR reaction. PCR is performed using the conditions described by Jepson *et al.* (1991). Plants transformed with GST-27 DNA are analysed with the primer GST II/7 (AACAAGGTGGCGCAGTT) (SEQ ID No. 10) specific to the 3' region of GST-27 region and NOS 3 (CATCGCAAGACCGGCAACAG) (SEQ ID No. 11) specific to the NOS terminator. 39 of the 42 primary transformants provide a 310 bp fragment by PCR.
**Western blot analysis.** To verify the heterologous expression of GST-27 in tobacco Western blot analysis is performed. 120 mg of leaf from 3-4 weeks old plants grown in sterile conditions are ground at 4°C in 0.06 g of polyvinylpoly-pyrolidone (PVPP) to adsorb phenolic compounds and in 0.5 ml of extraction buffer (1 M Tris HCl, 0.5 M EDTA (ethylenediamine-tetraacetate), 5 mM DTT (dithiothreitol), pH 7.8). An additional 200 µl of extraction buffer is then added. The samples are mixed and then centrifuged for 15 minutes at 4°C. The supernatant is removed, the concentration of protein being estimated by Bradford assay using BSA as the standard. The samples are kept at -70°C until required.
   Samples of 5µg of protein with 33% (v/v) Laemmli dye (97.5% Laemmli buffer (62.5 mM Tris HCl, 10% w/v sucrose, 2% w/v SDS, pH 6.8), 1.5% pyronin y and 1% - mercaptoethanol) are loaded on a SDS-polyacrylamide gel (17.7% 30:0.174 acrylamide:bisacrylamide), after 2 minutes boiling. Protein extracts are separated electrophoretically in the following buffer (14.4% w/v glycine, 1% w/v SDS, 3% w/v Tris Base). Then they are transferred onto nitro-cellulose (Hybond-C , Amersham) using an electroblotting procedure (Biorad unit) in the following blotting buffer (14.4% w/v glycine, 3% w/v Tris Base, 0.2% w/v SDS, 20% v/v methanol) at 40 mV overnight.
   Equal loadings of proteins are checked by staining the freshly blotted nitrocellulose in 0.05% CPTS (copper phtalocyanine tetrasulfonic acid, tetrasodium salt) and 12 mM HCl. Then the blots are destained by 2-3 rinses in 12 mM HCl solution and the excess of dye removed by 0.5 M NaHCO₃ solution for 5-10 minutes followed by rinses in deionised water. Filters are blocked for 1 hour with TBS-Tween (2.42% w/v Tris HCl, 8% w/v NaCl, 5% Tween 20 (polyxyethylene sorbitan monolaureate), pH 7.6) containing 5% w/v BSA. Then they are washed for 20 minutes in TBS-Tween supplemented with 2% w/v BSA. Indirect immunodetections are performed with a 1:2000 dilution of a sheep GST-27 antiserum as first antibody and with a 1:1000 dilution of a rabbit anti-sheep antiserum as second antibody, associated with the horseradish peroxidase (HRP). Any excess of antiserum is washed with TBS-Tween supplemented with 2% w/v BSA. ECL (enhanced chemiluminescence) detection is performed using the protocols described by Amersham. Any background is eliminated by additional washes of the membranes in the solution mentioned above.
   An estimation of the level of expression of the GST gene is performed on the LKB 2222-020 Ultroscan XL laser densitometer (Pharmacia). Western analysis reveals 8 of the PCR positive primary transformants show no detectable GST-27 expression. The remaining 31 show expression levels which vary from barely detectable to high levels equating to 1% of total soluble protein as determined from signals detected with pure maize GST II samples.
**Southern blot analysis.** The pattern of integration of transgenes is verified by Southern blot analysis. 2.5 g of fresh tobacco leaf taken from plants grown in glasshouse, placed into a plastic bag containing 0.75 ml of extraction buffer (0.35 M sorbitol, 0.1 M Tris HCl, 0.005 M EDTA, 0.02 M sodium meta bisulphite, pH 7.5), are crushed by passing through the rollers of a "Pasta machine". Crushed extracts are then centrifuged for 5 minutes at 6000 rpm at room temperature. After discarding the supernatant, the pellet is resuspended in 300µl extraction buffer and 300µl nuclei lysis buffer (2% w/v CTAB), 0.2 M Tris HCl, 0.05 M EDTA, 2 M Nacl, pH 7.5). 120µl of 5% Sarkosyl is added and the samples placed in a 65°C water bath for 15 minutes. Extracts are centrifuged for 5 minutes at 6000 rpm after adding 600 µl 24:1 chloroform:isoamyl alcohol. 700 µl of isopropanol is added to the same volume of supernatant and centrifuged for 10 minutes at 13,000 rpm. Then the pellet is washed with 70% ethanol and left to air dry. The pellet was left overnight at 4°C in 30 µl TE (10 mM Tris HCl, 1 mM EDTA) to resuspend. Samples are kept at -20°C until required.
   Total leaf DNA is digested for 6 hours at 37°C with the following restriction enzymes *Sac*I and *Xba*I in 1 x Phor-one-all buffer (20 mM Tris acetate, 20 mM magnesium acetate, 100 mM potassium acetate, Pharmacia) for the extracts from the plants containing the GST-27 gene. DNA is fractionated on a 0.8% agarose gel, denatured by gently shaking in 0.5 M NaOH, 1.5 M NaCl for 30 minutes and the gel is neutralized by shaking in 0.5 M Tris HCl, 1.5 M NaCl for 75 minutes. Then the DNA is transferred onto an Hybond-N (Amersham) nylon membrane by capillary blotting in 20 x SSC (3M Nacl, 0.3M Na₃citrate). DNA is fixed to membranes using a combination of UV strata linking (Stratagene) and baking for 20 minutes at 80°C. Probes are excised from plasmids, used for *Agrobacterium* transformation, containing the GST-27 gene by digestion with *Eco*RI. The probe is labelled with α³² - P dNTP (3,000 Ci/mM) using the Prime-α-Gene kit (Promega), random priming protocol described by Feinberg and Vogelstein. Positive controls are prepared by digestion of pIJ21-3A with *Sac*I and *Eco*RI.
   Prehybridisations are performed in 5 x SSPE (0.9 M Nacl, 0.05 M sodium phosphate, 0.005 M EDTA, pH 7.7), 0.5% SDS, 1% w/v Marvel (dry milk powder), 200 µg/ml denaturated salmon sperm DNA for 3-4 hours at 65°C. Hybridizations are performed in the same buffer but without the last ingredient. Membranes are washed for 30 minutes at 65°C in 3 x SSC, 0.5% SDS, and twice in 1 x SSC, 0.1% SDS for 20 minutes prior to autoradiography at -70°C.
**HPLC assay.** To verify the GST-27 expressing plants show GST activity against herbicide substrates an in vitro herbicide assay is performed using HPLC. 1 g of leaf tissue is taken from 3-4 month old flowering tobacco plants growing in the glasshouse, and ground in liquid nitrogen and 7 ml of extraction buffer (50mM glycyl glycine, 0.5mM EDTA, 1 mM DTT, pH 7.5). Extracts are transferred to centrifuge tubes containing 0.1g of PVPP and centrifuged at 16,500 rpm for 30 minutes at 4°. 2.5ml of supernatant is loaded onto Sephadex G-25 (PD10) column (Pharmacia) and eluted with 3.5ml of sodium phosphate buffer (50 mM, pH 7.0) containing 1 mM EDTA and 1 mM DTT. Protein estimation is performed by the Bradford method using BSA as the standard. Extracts are divided into aliquots and kept at -70°C until required. HPLC assays are performed on a Spherisorb 5µ ODS2 column (25 cm * 4.6 mm i.d., manufacturer: Hichrom) using 65:35 acetonitrile:1% aqueous phosphoric acid mobile phase at the rate of 1.5 ml/min. Detection of the compounds is performed on a UV LC-6A Schimadzu detector (wavelength 200 nm).
   Reactions are carried out in 0.8 ml HPLC vials at room temperature (20-25°C). 15-94% by volume of plant extract are added to the sodium phosphate buffer (pH 7), 5 mM glutathione or homoglutathione and 2 or 20 ppm of compound (2 ppm for fluorodifen, 20 ppm for acetochlor, alachlor and metolachlor). Controls are also set up in the same proportions but extracts replaced by the sodium phosphate buffer. Reactions are initiated by addition of the herbicide used as substrate. Compound reactivity is monitored for a maximum of 9-19 hours. Specific retention times and peak areas are calculated by the JCL 6000 chromatography data system package (Jones chromatography). HPLC peak area versus time profiles, based on 7-11 time points, are measured for each compound. Half-life and pseudo first-order rate constant data are obtained from exponential fits of corrected peak area versus time data. These data are mastered with the FIT package version 2.01.
   Using the methodology described above, the GST activity of the transformed plants is assayed against different herbicide substrates. These herbicides consist of 3 dichloroacetanilides (acetochlor, alachlor, metolachlor) and a diphenyl ether (fluorodifen). These chemicals are known to be conjugated to glutathione, in particular dichloroacetanilides. Extractions are performed in the presence of PVPP and at low temperature to limit denaturation of proteins. Studies on GST stability show that maize GST activity is reduced by 73% in crude extracts when stored at -20°C. Therefore it was decided to divide extracts in aliquots. They were kept at -70°C until required. Each sample was defrosted only once, overnight on ice. The assay is performed within 2 weeks following the extraction.
   Concentrations of herbicide in the HPLC vials are set according to their solubility limits. Acetochlor, alachlor and metolachlor were assayed at 20 ppm and fluorodifen at 2 ppm. The assay is run for 9-19 hours according to the reactivity of the herbicide. Metolachlor is assayed for a longer period of time, because its half-life is high under these conditions. Detection of the compounds is performed on a UV detector at 200nm. Specific retention times and peak area are monitored for the herbicide. The GST activity is calculated on the basis of 7-11 time points. Enzymatic conjugation follows an exponential decrease curve. The decrease of the peak area of the assayed herbicide is used for the calculation of the GST activity. The half-life and the first order rate constant are also calculated.
   Five tobacco lines are assayed including a wild-type (negative control), 4 GST-27 lines 5, 6, 12 and 17. They are chosen because of their high expression as determined by western analysis. To limit any rapid conjugation before monitoring, the herbicide is added last. The GST-27 line 17 is also assayed for conjugation of acetochlor to homoglutathione. Results are reported in Figure 7 and show GST-27 expressing plants exhibit activity against chloroacetanilide herbicides *in vitro.*
   In summary: transgenic tobacco plants express the GST-27 protein and these plants may be distinguished by their relative activities *in vitro* against herbicide substrates.
***In vivo* analysis - Rooting assay.** The GST-27 lines have significant activity *in vitro* against at least 3 chloroacetanilides. Moreover, most of the herbicides of this class are known to inhibit root elongation. Therefore, it is decided to set up a rooting test on acetochlor, alachlor and metolachlor.
   A pilot experiment is set up to find out the most effective concentrations. A range of 7 concentrations is chosen: 0, 1, 5, 10, 20, 40 and 100 ppm. Two transformed lines (GST-27 lines 6 and 17) and a wild-type tobacco are tested on alachlor. Lines 6 and 17 are chosen because they represent the lowest and the highest expressing plants, based on western blot analysis. Three explants, consisting of a leaf attached to a piece of shoot, are transferred onto MS medium supplemented with the herbicide. Root growth is observed after 2 weeks (Figure 8). On the general aspect of the plants, an effect of the herbicide is observable on the wild-type from the concentration 1 ppm, the leaves are more yellowish and smaller. With the increase of the concentrations, these effects are greater and the number of new leaves is reduced. From 10 ppm, the plants do not produce new leaves. In contrast, with respect to the transformed lines, the effect of the herbicide is observable from the concentration 20 ppm for line 2 and 40 ppm for line 6. Between these concentrations, the leaves seem smaller and their number slightly reduced, but they still are green. Secondly, the wild-type produces some roots up to 5 ppm, but their length decreases dramatically between the concentrations 0 and 5 ppm. Regarding the lines 2 and 6, roots are respectively produced up to 10 ppm and 20 ppm, with the decrease of their length for lower concentration. Under these conditions and after 2 weeks, it is noticeable that the concentration limiting the rooting is between 20 and 40 ppm for the "best" line tested at this stage of this experiment.
   A subsequent experiment is set up for a wild-type (control), 4 GST-27 (lines 5, 6, 12 and 17). These plants are assayed on acetochlor, alachlor and metolachlor at the following rates: 0, 10, 20, 40 ppm for the acetochlor and metolachlor mentioned herbicide, and 0, 20, 40, 100 ppm for alachlor. These concentrations are chosen because on HPLC the plants show the lowest activity against acetochlor and metolachlor. The same conditions are used: 3 explants per concentration and per line transferred onto MS medium supplemented with herbicide. The observations of the root growth are taken 3 weeks after the beginning of the assay.
   As for the pilot experiment the response of the explants in each pot is generally uniform. On acetochlor, the wild-type explants do not show any rooting or any production of new leaves in the presence of herbicide. But the GST-27 lines 6 and 17 produce few roots at 10 and 20 ppm and small leaves as well. The lines 5 and 12 are not as resistant as these 2 lines. On alachlor, the wild-type does not produce any root for the tested rates, but some leaves at 20 ppm. Lines 6 and 17 produce roots up to the concentration of 40 ppm, which roots appear not to be affected by the herbicide. The number of roots seems lower with increasing concentrations of herbicide. For these lines, the rooting concentration limit is between 40 and 100 ppm under these conditions and after 3 weeks. Lines 9 and 16 do not produce any roots but very tiny leaves at 20 and 40 ppm of the herbicide. On metolachlor, the wild-type tobacco produces very few tiny roots at 10 and 20 ppm. Lines 6 and 17 produce short roots, but not as many as are produced on alachlor. For this herbicide, the rooting concentration limit is between 20 and 40 ppm for the line 6 and more than 40 ppm for line 17.
**Treatment of plants with Herbicide.** To demonstrate that transgenic plants expressing GST-27 confer resistance to herbicide treatment, pre- and post-emergence herbicide trials are performed in the glasshouse.
   Pre-emergence tests are performed by sowing approximately 50 seeds per line for each rate of herbicide in sand (25 % sifted loam, 75 % grift, slow release fertiliser). Four replicates are treated for each chemical rate. Herbicide (0, 300 and 350 g/ha), formulated in 5 % JF 5969 (905.6 g/L cyclohexanone, 33.33 g/L synperonic NPE1800 and 16.7 g/L Tween 85) are applied to seed trays using a tracksprayer. Seeds are left to germinate in the glasshouse and germination is scored after 3 weeks. Results for alachlor show that the transgenic plants are resistant to the pre-emergent application of the herbicide. Similar results are obtained for acetochlor, metolachlor and EPTC (12000g/ha).
   Post-emergence tests are carried out by sowing 28 seeds per line and per herbicide rate in compost. After 16 days tobacco plants (1 cm high) are sprayed with alachlor in 5 % formulation JF 5969 using a tracksprayer. Damage is scored 3 weeks following spray treatment using size of the plants, necrosis, apex condition, morphology of leaves relative to unsprayed control. A score of 100 % damage means that the plant is killed by the herbicide and a score of 0% means that the plant resembled an untreated control. Post-emergent results for alachlor demonstrate that the transgenic plants are resistant to this herbicide. Damage to wild type plants and a segregating GST-27 line, is recorded graphically in Figure 9 following metolachlor treatment at 1400 g/ha. Similar studies are performed with acetochlor at 2000 g/ha giving similar results.

### EXAMPLE 4

### Cloning of glyphosate resistance genes into plant material and the generation of glyphosate resistant plants

A summary of the cassettes and specific plant transformation constructs used in this example is shown in the Figures of European Patent Application No. EP A1 536330.
**Dicot Vector 1** Vector 1 is a constitutive control plasmid containing the glyphosate oxidase gene (GOX) fused to the chloroplast transit (CTP) sequence 1 from the Rubisco gene of *Arabidopsis* driven by the enhanced 35S CaMV promoter. The construct contains the omega translational enhancer 5' of the CTP encoding sequence. Vector 1 utilises the NOS terminator. The CTP-GOX construct is synthesised to according to the sequence disclosed in WO92-00377 with the addition of an *Nco* I site at the translation start ATG, and a *Kpn* I at the 3' end. Internal *Sph* I sites and *Nco*I site are deleted during synthesis with no change in the protein sequence. The CTP-GOX sequence is isolated as an *Nco* I *Kpn* I fragment and ligated using standard molecular cloning techniques into *Nco I Kpn* I cut pMJB1, a plasmid based on pIBT 211 containing the CaMV 35 promoter with duplicated enhancer linked to the tobacco mosaic virus translational enhancer sequence which replaces the tobacco etch virus 5' non-translated leader, and terminated with the NOS terminator.
   A cassette containing the enhanced CaMV35S promoter-Omega enhancer- CTP-GOX-Nos sequence is isolated as a *Hind* III *EcoRI* fragment and ligated into *Hind* III *Eco*RI cut pJRIi, a pBin 19 based plant transformation vector.
**Dicot Vector 2.** The CP4- EPSPS (which is a class II EPSPS) fused to a chloroplast transit peptide from Petunia is synthesised according to the sequence depicted in WO92-04449 with an *Nco*I site at the translation initiation ATG. An internal *Sph* I site in the EPSPS is silenced with no change in protein sequence. A fragment containing the synthetic CTP-EPSPS sequence is isolated as a *Nco*I *Sac*I fragment and ligated into pMJBI. This sequence is placed under expression control of an enhanced 35S promoter and NOS terminator with an Omega fragment being positioned 5' of the protein encoding regions and isolated as an *EcoRI Hind* III fragment which is cloned into pJRIi to give dicot vector 2.
**Dicot Vector 3**. A control vector with both EPSPS and GOX genes is constructed by cutting dicot vector 2 with *EcoRI* and inserting an *EcoRI - Sph* I - *EcoRI* linker. The resultant vector is cut with *Sph* I to liberate a cassette ("B"), which is cloned into an *Sph* I site in dicot vector 1, 5' to the promoter to form pDV3puc (Figure 9). The coding regions, including promoters and terminators derived from vectors (1) and (2) are then excised from pDV3puc as a *Hind* III and *EcoRI* fragment and cloned in to pJRIi .
   Plasmid pDV3 in the binary vector pJR1i is introduced into tobacco by *Agrobacterium* mediated transformation using known techniques. 270 Shoots are removed from calli obtained from the thus transformed material, 77 of which rooted. To confirm the presence of the EPSPS and GOX genes in the thus rooted shoots, DNA extracts are prepared from pDV3 plants and analysed by PCR using the following primers:
   3' end EPSPS gene
      GATCGCTACTAGCTTCCCA (SEQ ID No. 12) EPSPS 2
   5' end GOX gene
      AATCAAGGTAACCTTGAATCCA (SEQ ID No. 13) GOX 1

PCR reactions provide a 1.1 kb band if both genes are present. To confirm the functionality of the glyphosate tolerance genes pDV3 tissue culture explants are transferred to MS media containing 0.01 mM and 0.05mM glyphosate. Plants are scored two weeks following transfer to medium containing glyphosate. Resistant lines, which grow successfully on herbicide-containing media, are analysed by Western using anti-sera raised in rabbits against purified GOX and EPSPS.

Leaf DNA extracts are prepared from each primary transformant and used for PCR reactions to confirm the presence of the vector. Western blot analysis is performed on each PCR positive pDV3 plant to verify the heterologous expression of GOX and EPSPS, using the methods described earlier. High level expressors are self- pollinated and seed screened on kanamycin plates. Single locus plants are kept for homozgote production. Data confirming that plants transformed with the pDV3 construct are resistant to glyphosate is to be found in Example 8.

### EXAMPLE 5

### Production of plants which are resistant to anilide type herbicides and glyphosate

Heterozygous and homozygous tobacco lines expressing GOX and EPSPS are cross-pollinated onto homozygous tobacco lines expressing GST-27. The seed generated in this cross are sown and leaf material taken for western analysis, using the procedures described earlier. Protein extracts from GST-27 western positive plants are then screened with the GOX/EPSPS antibody to select lines expressing both GST-27, GOX and EPSPS. These lines are then used in pre-emergent herbicide sprays with acetochlor, alachlor, metoloachlor and EPTC. Subsequently, the plants can be sprayed in a post-emergent manner with formulated glyphosate.

### EXAMPLE 6

### Production of plants which are resistant to both anilide and glyphosate type herbicides by a process not involving cross-pollination

The vector pDV3puc is cut with EcoRI, phenol chloroform extracted and precipitated. A delta EcoRI- HindIII- EcoRI linker MKOL3 5'AATTACGGAAGCTTCCGT3 ' (SEQ ID No.14) is heated to 70°C and cooled to room temperature allowing it to self-anneal: The annealed linker is then ligated into *Eco*RI cut pDV3puc. Putative recombinants are screened with end labelled oligonucleotide MKOL3. Plasmid DNA is isolated from positively hybridising colonies. Restriction digestion with HindIII release a 5.4 kb fragment containing the 35S CaMV promoter driving expression of Omega-CTP2- EPSPS- NOS and the 35S CaMV promoter driving expression of Omega-CTP1-GOX-NOS. This fragment is cloned in to pGST-27 Bin cut with *Hind*III and dephosphorylated with CIP. Recombinants are selected using an insert probe. The resultant vector pDV6-Bin (Figure 10) is verified by appropriate sequence analysis The resultant plasmid is transformed into Tobacco via *Agrobacterium* using known techniques. 270 Shoots are recovered following transformation, 80 of which are rooted. Leaf DNA extracts are prepared from each primary transformant and are used in PCR reactions to confirm the presence in the leaf of the protein encoding regions of the vector. The primers are as indicated above (SEQ ID Nos. 12 and 13). To confirm the functionality of the trans-genes, primary transformants from pDV6-Bin are assessed on 0, 0.01mM an 0.05mM glyphosate and 10ppm and 40ppm alachlor in tissue culture medium. A number of transgenic grow successfully on both media under conditions in which the wild type controls fail to. Western blot analysis is performed on each PCR positive plant to verify the heterologous expression of GOX and EPSPS and GST-27, using the methods described earlier. These lines are then used in pre-emergent herbicide sprays with acetochlor, alachlor, metoloachlor and EPTC. Subsequently, the plants can be sprayed in a post-emergent manner with formulated glyphosate.
Table 1 below gives the data for the pre-post herbicide treatments of DV6 plants ie plants expressing both glyphosate resistance genes and GST. The top half of the table shows the rates at which the pre-em herbicides are applied and their continued state in the absence of post-em herbicide application. The lower half of the top table gives the damage incurred after a glyphosate treatment of 800g/ha. The lower table shows the replicate scores for damage inflicted on the plants not subjected to a pre-em treatment as a result of the post-em glyphosate treatment. All replicates of the wild type plants score similarly whereas the transgenic scores reflect the fact that this was a segregating population ie azygous plants not expressing transgenes are able to go through to the post-em spray test.

**Table 1 MEAN DATA FOR POST EM HERBICIDE TREATMENT 21 DAT**

| Post treatment | | Pre treatment | | | % Phytotoxicity | |
|---|---|---|---|---|---|---|
| Chemical | Rate | Chemical | Rate | Pdv6 #2 | pDV6 #71 | Wild type |
| None | | None | - | 0 | 0 | 0 |
| | | Acetochlor | 50 | 0 | 0 | - |
| | | Metolachlor | 300 | 0 | 0 | - |
| | | Alachlor | 400 | 0 | 0 | - |
| | | Dimethenamid | 50 | 0 | 0 | - |
| | | Cycloate | 5000 | 0 | 0 | - |
| | | EPTC | 5000 | 0 | 0 | - |
| | | Bayer FOE 5043 | 50 | 0 | 0 | - |
| | | Tetrazolinone | 200 | - | 0 | |
| Glyphosate | 800 | None | - | 18.75 | 48.75 | 86.25 |
| | | Acetochlor | 50 | 0 | 0 | - |
| 360 g/l | | Metolachlor | 300 | 0 | 0 | - |
| | | Alachlor | 400 | 0 | 0 | - |
| | | Dimethenamid | 50 | 0 | 0 | - |
| | | Cycloate | 5000 | 0 | 0 | - |
| | | EPTC | 5000 | 0 | 0 | - |
| | | Bayer FOE 5043 | 50 | 0 | 0 | |
| | | Tetrazolinone | 200 | - | 0 | - |

| Post treatment | | Pre treatment | | | | |
|---|---|---|---|---|---|---|
| Chemical | Rate | Chemical | a | b | c | d |
| Glyphosate | 800 | None | 75 | 0 | 0 | 0 |
| | | | | | | |
| | | | 100 | 0 | 0 | 95 |
| | | | | | | |
| | | | 90 | 90 | 90 | 75 |

| | | | | | | |
|---|---|---|---|---|---|---|
| FOE 5043 is an oxyacetamide known as fluthiamide. | | | | | | |

### EXAMPLE 7

### Production of maize which is resistant to glufosinate and anilide type herbicides.

A monocotyledonous (maize, wheat) transformation vector containing GST-27, conferring resistance to pre-emergence herbicides, and phosphinothricin acetyl transferase (PAT), conferring resistance to the post-emergence herbicide glufosinate is generated as follows:
Step 1: Digest pUB 1 (a pUC based vector containing the maize ubiquitin promoter and intron) (Figure 11) with *Hind* III. Into the gap produced by the digestion is inserted a *Hin*dIII*-Age* I-*Hin*dIII linker (5' AGCTTGTACACCGGTGTACA 3' (SEQ ID No. 15)). The result recombinant vector is designated as pUB2.
**Step 2:** The GST-27 cDNA is excised from pIJ21-3A using *Kpn* I and *Bam*HI and cloned into *Bam*HI and *Kpn*I cut pUB2 to form pUB3.
**Step 3:** A *Kpn*I*-Pac*I*-Kpn*I linker (5'CGGACAATTAATTGTCCGGTAC 3' (SEQ ID No. 16)) is self annealed and cloned into *Kpn*I cut pUB3 to form pUB4.
**Step 4:** The NOS terminator is isolated as a *Sma*I fragment from pIE98 (Figure 12), and blunt end cloned into *Eco*RV cut pUB4 to form pUB5. The orientation of the NOS terminator in pUB5 is confirmed by restriction digestion with *Eco*RI and *Bam*HI. All junctions are sequencesd to confirm the correct insertion of the various construct components.
**Step 5:** The ubiquitin GST-27 NOS cassette present in pUB5 is removed from it by digestion with *Age* I and *Pac*I and is cloned in the ampicillin minus vector pIGPAT (Figure 13) which contains the PAT gene under the control of the 35S-CaMV promoter. Recombinants are detected by colony hybridisation with an *Eco*RI cDNA insert from pIJ21-3A. Recombinants are detected by colony hybridisation with an EcoR1 cDNA insert from pIJ21-3A. Recombinants are orientated with *Nco* I restriction digestion to form pCAT10 (Figure 14).
**Step 6:** The 35S-PAT-NOS cassette is removed by digestion with AscI and the AscI ubiquitin-PAT-NOS cassette from pPUN 14 inserted to form pCAT11 (Fig 15). pCAT11 is transformed into wheat and maize using known whiskers and particle bombardment technology. The cells are then transferred into bialophos-containing media to select callus material which expresses the PAT gene. Calli which grows on media containing this herbicide are then subjected to PCR using the following primers (SEQ ID Nos. 33 and 34 respectively) to conform the presence in the calli of the GST-27 gene.
   5'CCAACAAGGTGGCGCAGTTCA3' (SEQ ID No. 33)
   5'CATCGCAAGACCGGCAACAG3' (SEQ ID No. 34).

The calli which contain the GST-27 expression cassette are transferred to plant regeneration media and maize plants are recovered. The transformed maize plants are confirmed - by Western blots of total protein extracts from leaves - to constitutively express the GST gene at high levels. Such plants are cross pollinated with an elite maize inbred line and seed is recovered. To confirm enhanced tolerance of the plants to the herbicide acetochlor the said seeds are planted in soil to which has been applied between 2,000 and 8,000 grams per hectare of the herbicide. The seeds are allowed to germinate and grow for 7 days after which time a sample of the resultant seedlings is assessed for damage caused the chemical and compared to the seedlings (if any) which result from non-transgenic seed sown under identical conditions. The "transgenic" seedlings and non-transgenic control seedlings grown in soil treated with the herbicide and a corresponding safener exhibit little, if any damage, whereas non-transgenic seedlings grown in soil which contains herbicide in the absence of safener show very substantial damage. Seedlings which survive the first herbicide treatment are allowed to grow for a further 20 days or so, and then sprayed with a commercial mix of glufosinate at various concentrations ranging from about 0.1 to 1% active ingredient. The seedlings which contain the PAT gene (expression of which is determined by the method described by De Block M.*et* (The EMBO Journal 6(9): 2513-2518 (1987)) are either completely resistant to glufosinate, or are relatively tolerant of the herbicide - depending upon the concentration applied - when compared with seedlings which do not contain the said gene.

### EXAMPLE 8

### Production of plants (mono and dicots) which are resistant to both glyphosate and glufosinate

This example demonstrate the production of plants which are resistant to both glufosinate and glyphosate. This multiple herbicide resistance results from the crossing of a first plant which has been engineered to be resistant to glufosinate with a second plant which has been engineered to be resistant to glyphosate.

### Production of a Glufosinate resistance construct pPG6

pPG6 is a Bin 19 based vector derived from pBin19RiPAT, and contains a cassette containing the 35S CaMV promoter driving the GUS gene. Inserted between the promoter and GUS is the second intron of the ST-LS1 gene. This sequence is 189bp, has an A/T content of 80%, typical splice junctions and stop codons in all three reading frames. The presence of the intron prevents expression of GUS in *Agrobacterium* as splicing does not occur in prokaryotes. It also contains a cassette carrying the 35S CaMV promoter driving expression of the PAT gene. Fig 16 shows a map of pPG6.

### Glyphosate resistance constructs

Dicot vectors 1-3 are produced as indicated above in Example 4.
**Monocot vector 1** Monocot vector 1 is a plasmid containing both CTP1 GOX and CTP2 EPSPS, both driven by the maize polyubiquibitin promoter and enhanced by the maize polyubiquitin intron 1, in a pUC derived plasmid. It also contains a cassette conferring tolerance to phosphinothricin.
   Plasmid 1: The vector pUB1 is digested with Kpn1 and a Kpn1-Not1-Kpn1 linker inserted, (sequence 5' CAT TTG CGG CCG CAA ATG GTA C 3 - SEQ ID NO. 17). An EcoR1-Not1-EcoR1 linker (5' AAT TCA TTT GCG GCC GCA AAT G 3' (SEQ ID No. 18) is inserted into the EcoR1 site of DV1-pUC. The resulting plasmid is cut with Nco1 and the 5' overhang filled using DNA Polymerase 1 Klenow fragment. The linear vector is then digested with Not1 and a Not1-blunt fragment isolated. This fragment, containing the CTP1-GOX and NOS sequences is ligated into Sma1-Not1 digested modified pUB1. A Hind111-Not1-Hind111 linker (sequence 5' AGC TTG CAG CGG CCG CTG CA 3' (SEQ ID No. 19)) is inserted into the plasmid to give resulting plasmid 1.
   Plasmid 2: An EcoR1-Not1-EcoR1 linker (5' AAT TCA TTT GCG GCC GCA AAT G 3' (SEQ ID No. 20)) is inserted into the EcoR1 site of DV2-pUC (another clone is isolated which does not contain the linker mentioned above, thus allowing this cloning strategy).The resulting plasmid is digested with Nco1 and the 5' overhang filled using DNA Polymerase 1 Klenow fragment. The linear vector is then cut with Not1 and the resulting fragment is cloned into the same vector as described immediately above(pUB1 modified), to generate plasmid 2. The PAT selectable marker cassette, comprising 35S CaMV promoter, Adh1 intron, phosphinothricin acetyl transferase (PAT) gene and nos terminator is excised from pIE108 and cloned into the Hind111 site of plasmid 2 to give cassette 2. Diagnostic restriction analysis is used to confirm that the PAT cassette was in the same orientation as the CTP2 EPSPS cassette.
   The cassette carrying the polyubiquitin promoter and intron, CTP1 GOX and nos terminator is excised from plasmid 1 on a Not1 fragment and ligated into Not1 cut cassette 2 to give monocot vector 1, pMV1 (Fig. 17).
**Tobacco transformation** Plasmids for dicot transformation are transferred to *Agrobacterium tumefaciens* LBA4404 using the freeze thaw method of Holsters et al (1978). *Nicotiana tabaccum var Samsun* is transformed using a leaf disc method described by Bevan et al (1984). Shoots are regenerated on medium containing 100mg/l kanamycin. After rooting and selection plants are transferred to the glass house and grown under 16hr light 8hr dark regime. Transformants of pPG6 are named as 35S-PAT lines.
Maize transformation Maize transformation is performed using the particle bombardment method as described by Klein et al (1988). Selection of the transformed material is on 1mg/l bialophos.

### PLANT ANALYSIS

PCR This analysis is performed on all tobacco lines which rooted in tissue culture and maize calli. DNA is extracted by means known to the skilled man. The primary transformants are analysed using the following oligonucleotides:-

| | | |
|---|---|---|
| pDV1 | TMV1 + GOX1, | GOX3 + nos1 |
| pDV2 | TMV1 + EPSPS1, | EPSPS1 + nos1 |
| pDV3 | EPSPS3 + GOX3 | |
| pPG6 | 35S + BARJAP2R | |
| pMV1 | GOX 4 + GOX5 | EPSPS4 + EPSPS5 35S + BARJAP2R |

The sequences of the oligonucleotides are:-

| | |
|---|---|
| TMV1 | 5' CTCGAGTATTTTTACAACAATTACCAAC (SEQ ID No. 21) |
| GOX1 | 5' AATCAAGGTAACCTTGAATCCA (SEQ ID No. 22) |
| GOX3 | 5'ACCACCAACGGTGTTCTTGCTGTTGA (SEQ ID No. 23) |
| nos1 | 5' GCATTACATGTTAATTATTACATGCTT (SEQ ID No. 24) |
| EPSPS1 | 5' GTGATACGAGTTTCACCGCTAGCGAGAC (SEQ ID No. 25) |
| EPSPS3 | 5' TACCTTGCGTGGACCAAAGACTCC (SEQ ID No. 26) |
| EPSPS4 | 5' ATGGCTTCCGCTCAAGTGAAGTCC (SEQ ID No. 27) |
| EPSPS5 | 5' CGAGACCCATAACGAGGAAGCTCA (SEQ ID No. 28) |
| GOX4 | 5' ATTGCGTGATTTCGATCCTAACTT (SEQ ID No. 29) |
| GOX5 | 5'GAGAGATGTCGATAGAGGTCTTCT (SEQ ID No. 30) |
| 35S | 5' GGTGGAGCACGACACACTTGTCTA (SEQ ID No. 31) |
| BARJAP2R | 5' GTCTCAATGTAATGGTTA (SEQ ID No. 32) |

| | |
|---|---|
| PCR +ve plants are selected for further analysis. | |

**Selection on glyphosate** A kill curve is constructed for growth of tobacco in tissue culture on glyphosate containing medium. This is done by inserting a stem segment ~6mm long and carrying a leaf node into MS medium containing a range of glyphosate isopropylamine concentrations. Four/five stem segments are used at each concentration. The results are scored after two weeks and are shown in Table 2.

**Table 2: Kill curve of glyphosate on wild type tobacco**

| Glyphosate isopropylamine conc'n(mM) | Growth of explant |
|---|---|
| 0 | Good stem growth, 4-5 new leaves, roots~5cm |
| 0.005 | No growth in any organ |
| 0.011 | " |
| 0.0275 | " |
| 0.055 | " |
| 0.1 | " |

Primary transformants of pDV1,2 and 3 are selected by growing on medium containing 0.01 and 0.05 mM glyphosate isopropylamine salt as described above. The results are shown in Table 3.

**Table 3:Selection of glyphosate tolerant lines in tissue culture**

| | pDV1 | pDV2 | pDV3 |
|---|---|---|---|
| tested on herb | 50 | 25 | 50 |
| tolerant lines | 25 | 18 | 20 |

**Selection on PAT** Regenerating calli are tested on 1mg/l bialophos.
Western analysis Over expression of GOX and EPSPS proteins and antibody generation are performed by means known to the skilled man. Tobacco primary transformants are analysed as follows. ~100mg PVPP is added to the bottom of an Eppendorf tube. Leaf material (four leaf discs obtained by using the tube lid as cutter) are harvested onto ice. 0.5ml extraction buffer (50mM Tris Hcl pH 7.8, 1mM EDTA sodium salt, 3mM DTT) and 2µl 100mM PMSF is added. The samples are ground in a cold room using an electric grinder. Grinding is continued for 10s, unground material pushed back into the tube and grinding continued for another 10-15s until the sample is homogeneous. Tubes are centrifuged for 15' in the cold room, supernatants removed to fresh tubes and frozen at -70C until required. Protein concentrations are determined using the known Bradford method. 25µg protein are fractionated by SDS PAGE and blotted overnight at 40mA onto a Hybond-N membrane. The filter is removed from the blotting apparatus and placed in 100ml 1X Tris-Saline 5% Marvel and shaken at RT for 45'.The filter is washed by shaking at RT in 1X Tris -Saline 0.1% Tween 20- first wash 5', second wash 20'. The primary antibody is used at 1:10000 dilution in 1X Tris-Saline 0.02% Tween 20. The membrane is incubated with the primary antibody at RT 2 hours or over night at 4C. The membrane is washed in 1X T-S 0.1% Tween at RT for 10' then for 1hour. The second antibody (anti rabbit IgG peroxidase conjugate) is used at 1:10000 dilution, incubation with the membrane was for 1 hour at RT. Washing is as described above. Detection is performed using the Amersham ECL detection kit. A range of protein expression levels are observed in the pDV1 and 2 lines based on the Western results. Expression levels of GOX and EPSPS in the PDV3 showed little variation in the amount of GOX being expressed but increased variation in the,amount of EPSPS. Lines expressing both genes are selected for further analysis.
Maize calli are analysed in the same way, calli expressing GOX and EPSPS are regenerated into whole plants and leaf material analysed again for expression of both genes.
Phosphinothricin acetyl transferase activity assays PAT activity is measured using ¹⁴C labelled acetyl CoA. The labelled acetyl group is transferred to the phosphinothricin(PPT) substrate by the PAT in the leaf extracts. Acetylated PPT and ¹⁴C migrate at different rates on a TLC plate, and can be visualised by autoradiography. Leaf extraction buffer is prepared using 10X T₅₀E₂ buffer (TE)-50ml 1M Tris.HCl pH7.5, 4ml 0.5M EDTA and 46ml dd water. Leupeptin is made up at a rate of 15mg/ml in 1X TE. Stock PMSF is made up in methanol to 30mg/ml. BSA stock solution is made at 30mg/ml in TE, and DTT at 1M. PPT is used as 1mM solution in TE. ¹⁴C Acetyl CoA was 58.1mCi/mmol (Amersham). The extraction buffer is made by combining 4315µl dd water, 500µl 10X TE, 50µl leupeprin stock, 25µl PMSF stock, 100µl BSA stock and 10µl DTT stock (final volume 5000µl). Leaf samples are harvested into Eppendorf tubes on ice using the lid as cutter. The samples (three pieces) are ground in 100µl extraction buffer using an electric grinder in cold room. The samples are centrifuged for 10minutes and 50µl removed to a fresh tube on ice. Samples are stored at -70C until use. Bradford analysis is used to quantify the protein present in the extracts. The substrate solution is prepared by mixing 5 volumes of labelled Acetyl CoA with 3 volumes of 1mM PPT solution. To a ~25µg total leaf protein sample (~2µg/µl) is added 2µl substrate solution, the mixture is incubated at 37C for30", then removed to ice to stop the reaction. A sample of 6µl is spotted onto a silica gel TLC plate (Sigma T-6770). Ascending chromatography is performed in a 3:2 mix of isopropanol and 25% ammonia solution, for 3 hour. Plates are wrapped in plastic film and exposed o/n to Kodak XAR-5 film. All 26 primary transformants are assessed for PAT activity using this method of analysis. Table 4 below gives details of the result of this analysis.

**Table 4:PAT activity data**

| PAT activity | pPG6 line |
|---|---|
| High | 1,9,11,12,14,21,22,24,25,27,28,30,32 |
| Medium | 5,7,10,15,19,20 |
| Low | 6,2,8 |

**Herbicide leaf painting** 35S-PAT primary transformants showing a range of PAT activity and control plants are tested by painting of Challenge onto individual leaves. Both surfaces of marked leaves are painted with a 1% and 0.2% solution of the stock solution (150g/l) in water. Scoring is performed after 48 hr and one week and leaf samples are taken for PAT assay.Table 5 shows the results of leaf painting.

**Table 5:Leaf paint analysis**

| Expression | Plant line | 0.2% | 0.2% | 1% | 1% |
|---|---|---|---|---|---|
| | | 48hr | 1 week | 48hr | 1 week |
| High | 24,1,14 | Undamaged | Undamaged | Undamaged | Undamaged |
| Low | 6,10 | Undamaged | Dead | Dead | Dead |
| Wild type | | Dead | Dead | Dead | Dead |

### Herbicide spray test

**Glufosinate (Challenge or Basta)**. A dose response curve is established for the effect of Challenge on wild type tobacco. Five plants are used in each treatment, the scoring is performed after 14 days. Following construction of the kill curve, selected 35SPAT lines are subjected to spray tests using Challenge, at the same rates of application. Table 6 shows this data, for two lines, #12 and 27. Transgenic plants showed no damage at these rates.

**Table 6: Results of spray test on 35S primary transformants**

| *Basta rate* | *Wild type* | *35SPAT#12* | *35SPAT#27* |
|---|---|---|---|
| | %damage | %damage | %damage |
| 200g/ha | 30 | 0 | 0 |
| 400g/ha | 40 | 0 | 0 |
| 600g/ha | 40 | 0 | 0 |
| 900g/ha | 80 | 0 | 0 |

A kill curve is established for the effect of glyphosate on wild type tobacco. Wild type tobacco growing in tissue culture is sub-cultured by taking stem segments and growing in fresh medium to generate 20 new plants. These are grown in tissue culture for one month before transfer to 3inch pots in John Innes No 3 compost. They are initially covered in fleece to protect them. After uncovering they are allowed to acclimatise for four days before being sprayed. After spraying watering is only into the saucers i.e. no water is allowed to touch the leaves for five days. Scoring was done 8 days and 28 days after treatment. Table 7 shows the mean percentage damage (three reps per treatment) at a range of application concentrations.

**Table 7: dose response curve of wild type tobacco treated with glyphosate at the rates indicated.**

| Trt. | Compound | Rate g/ha | Adjuvant | Nicotiana wild type |
|---|---|---|---|---|
| No | | | | |
| 1 | Roundup Ultra (USA) | 100 | 'Frigate' | 73 |
| 2 | 480g/l glyphosate- | 200 | (K30512) | 90 |
| 3 | isopropylamine | 400 | | 99 |
| 4 | (a.e. 360g/l) | 800 | | 100 |
| 5 | LDJW010017 | 1200 | | 100 |
| 6 | | 1600 | | 100 |
| | | | | |

Following construction of the glyphosate kill curve, a number of pDV1,2 and 3 lines are spray tested with appropriate rates of glyphosate. Table 8 shows the results for pDV3, the results for pDV1 and pDV2 lines are similar to those of pDV3.

**Table 8: dose/response of pDV3 primary transformants treated with glyphosate**

| | | | | | | | |
|---|---|---|---|---|---|---|---|
| | 1 | 2 | 3 | 4 | 5 | 6 | 7 |
| Rate g/ha | 12.33 | 37 | 100 | 300 | 1000 | 3000 | 9000 |
| Wildtype | 2 | 3 | 20 | 80 | 93 | - | - |
| pDV3#11 | - | - | 0 | 0 | 25 | 70 | 80 |
| #14 | - | - | 7 | 22 | 13 | 33 | 76 |
| #19 | - | - | 0 | 0 | 0 | 35 | 80 |
| #21 | - | - | 0 | 5 | 0 | 24 | 78 |
| #31 | - | - | 12 | 0 | 4 | 26 | 78 |
| #34 | - | - | 0 | 0 | 6 | 30 | 63 |
| #36 | - | - | 0 | 0 | 0 | 0 | 0 |
| #37 | - | - | 0 | 0 | 9 | 24 | 72 |
| #43 | - | - | 0 | 0 | 0 | 6 | 73 |
| #44 | - | - | 0 | 40 | 45 | 78 | 85 |
| #45 | - | - | 0 | 0 | 3 | 9 | 70 |
| #47 | - | - | 5 | 0 | 0 | 11 | 72 |
| #60 | - | - | 0 | 0 | 0 | 19 | 63 |
| #64 | - | - | 0 | 5 | 0 | 12 | 63 |

Segregation analysis. Seed from each primary transformant (pPG6, pDV1, pDV2 and pDV3 is sterilised in 10% Domestos for twenty minutes. After several washes in sterile water, 100 seed of each selfed primary transformant is plated onto 0.5XMS (2.3g/l MS salts, 1.5% sucrose, 0.8% Bactoagar, pH 5.9)medium containing 100mg/l kanamycin. Seedlings are scored after three weeks growth at 26°C under16hr light/8 hr dark. Lines segregating in a ratio of 3:1 are assumed to have single transgene insertions. In the case of the pPG6 lines, #12, 20, 27 segregated in the desired ratio. In the case of the pDV3 lines, # 14,19,21,31,34,43 and 45 segregated in the desired ratio.
**Generation of homozygous lines** From the segregation tests 10 unbleached seedlings, (heterozygotes or homozygotes) are transferred to fresh medium in tubs and grown on for two-three weeks. After this time they are transferred to JI No 3 compost in 31 pots to flowering. Seeds are retested on Km containing 0.5XMS to identify homozygous lines.
**Crossing of tobacco lines** Homozygous lines containing pDV1, pDV2 and pDV3 ie plants expressing GOX, EPSPS and GOX/EPSPS genes respectively are cross pollinated onto a homozygous pPG6 line expressing the PAT gene, line # 27. The pollination is also performed using the pPG6 line as the male line.
**Analysis of transgenic corn lines** Regenerating calli are tested by PCR using the oligos described above ie 35S-AlcR, AlcA-GOX1, and internal oligo's for GOX and EPSPS. Western analysis is also performed on the PCR +ve calli to select those expressing GOX and EPSPS. Those calli are regenerated and the resulting plants are re-analysed by PCR. The plants are then backcrossed and selfed.

### Analysis of tobacco progeny

**GOX, EPSPS and PAT expression.** All progeny are homozygous for both genes. Seed from each crossing and seed from each parent homozygote is sown and leaf material harvested from a number of plants for analysis. Protein extracts are analysed by western blotting and then by PAT activity measurements as described previously. Levels of expression of GOX and EPSPS and PAT activity are found to be similar to each other within a particular cross and to those of the homozygote parent. The plants are scored for appearance, height, vigour of growth etc.
**Herbicide treatments.** Three broad experiments are designed:-
   1.35S-PAT cf pDV1 cf pDV1-PAT
   2.35S-PAT cf pDV2 cf pDV2-PAT
   3.35S-PAT cf pDV3 cf pDV3-PAT
   35S-PAT lines are treated with glufosinate at a range of concentrations and the rates at which particular degrees of damage occurred identified, at different time points. DV lines are treated with glyphosate at a range of concentrations and the damage rate identified. DV-PAT lines are then treated with mixtures of the two herbicides at different ratios and the level of damage assessed. Each of the populations are treated at the 5-6 leaf stage (5 reps per treatment).
**Resistance to pathogen attack** 35S-PAT, DV1,2 and 3 and DV-PAT lines expressing good levels of each protein and showing good herbicide tolerances are exposed to a number of fungal pathogens and the level of infection scored and compared.
Analysis of maize progeny. The seed resulting from the crossing of the primary transformants is used to generate plants from which to select the best expressing lines. This is done by western analysis of expression levels of GOX, EPSPS and by PAT activity experiments as described above. Similar experiments are performed to determine herbicide tolerance to glyphosate and glufosinate, either applied singly or in various combinations.

### EXAMPLE 9

### Production of plants tolerant to pre-emergent bleaching herbicides eg fluorochloridone, norflurazon, fluridone, flurtamone and diflufenican and to glyphosate.

Phytoene desaturase (PDS) inhibitors eg flurochloridone and norflurazon are a group of herbicides which block carotenoid biosynthesis and give rise to bleaching symptomology. The PDS gene (*crt1*) is cloned from E*rwinia uredovora,* a non-green phytopathogenic bacterial rot, and over-expressed in transgenic tobacco (and tomato)using a plasmid containing the CaMV 35S promoter and a chloroplast transit peptide (pYPIET4) (Misawa *et al*., 1993). Homozygous seed of line ET4-208 tobacco plant which over-expresses the *crtl* gene are obtained as are tomato plants containing the same construct.
**Herbicide tolerance trials** Compounds of formulas (I), (II) and (III) (see below) are tested. Transgenic and wild type tomato seed (cv Ailsa Craig) is sown in 3" pots of JIP 3, three seeds per pot. Each compound is formulated in 4% JF5969 (apart from compound I which is a commercial formulation) and sprayed onto the units in the track sprayer at 200 l/ha. The test is assessed at 13, 20 and 27 DAT (days after treatment). There are clear dose responses from all treatments on the wild type tomato, with the highest rate in all cases giving 87-100% phytotoxicity. The transgenic tomatoes are highly tolerant of all of the PDS inhibitors tested, at least to 1 kg/ha of compounds II and III and up to 9 kg/ha of compound I (see Table 9). Similar results are obtained for transgenic tobacco. 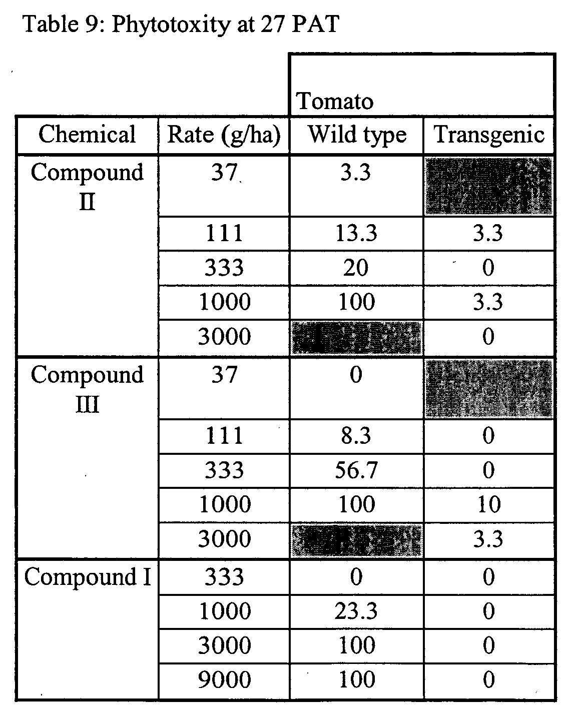 DV3 # 43B ( a glyphosate resistant line comprising the EPSPS and GOX genes - see Example 4) is cross pollinated onto homozygous ET4-208 and *vice versa* in the usual way. Seed is collected and used in herbicide trials similar to those described above. The tobacco seed is sown in rows in small units the day before treatment. Each compound is formulated in 4% JF5969 (apart from Racer which was a commercial formulation) and sprayed onto the units in the track sprayer at 200 1/ha. The test is assessed at 13, 20 and 27 DAT. Seedlings that are tolerant to bleaching herbicides are transferred after the final assessment into fresh John Innes 111 compost in 3" pots. After two weeks they are subjected to glyphosate herbicide applied at 500 and 800 g/ha. Scoring is performed 14 and 28 DAT. The resultant plants are resistant to both classes of herbicide, and the resistance is inherited in a Mendelian manner.

### Compound of Formula I:

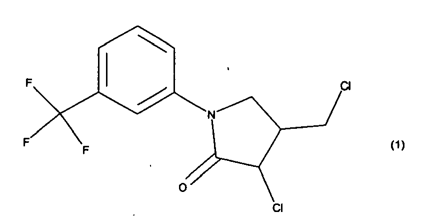

### Compound of Formula II:

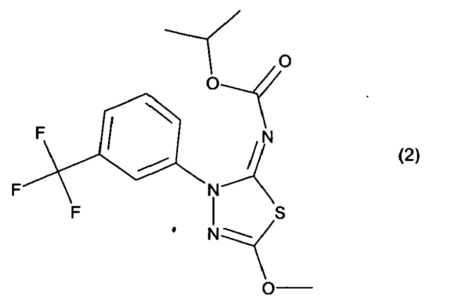

### Compound of Formula III:

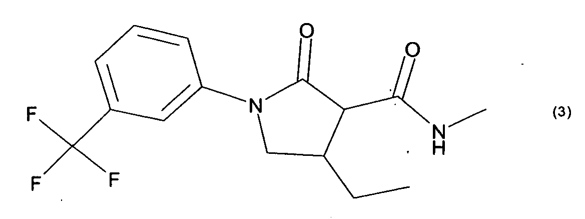

### EXAMPLE 10

### Generation of plants tolerant to triketones, acetanilides and glyphosate

pDV6 # 71 G and pDV3 # 19J are cross pollinated onto a homozygous triketone tolerant line and vice versa as described earlier. Seed are collected and used in herbicide trials as described below. The tobacco seeds obtained from the DV6/HPPD cross are sown in rows in small units the day before treatment. Some units are treated with acetochlor (75 g/ha), some with alachlor (300 g/ha) and others with ZA1296 (100 and 300 g/ha). Assessment is at 21 DAT. The scores are given below for the 21DAT assessment and represent phytotoxicity.

| | | Wild Type | Wild type | DV6/HPPD | DV6/HPPD |
|---|---|---|---|---|---|
| Chemical | Rate (g/ha) | Rep a | Rep b | Rep a | Rep b |
| Acetochlor | 75 | 40 | 100 | 0 | 0 |
| Alachlor | 300 | 80 | 90 | 0 | 0 |
| ZA1296 | 100 | 90 | 95 | 10 | 0 |
| | 300 | 100 | 100 | 0 | 0 |

Seedlings surviving the 300 g/ha treatment of ZA1296 are sprayed with 800 g/ha of glyphosate and demonstrate tolerance to this.

## Claims

1. A polynucleotide comprising at least a first region encoding a first protein capable of conferring on a plant, or tissue comprising it, resistance or tolerance to a first herbicide, and a second region encoding a second protein likewise capable of conferring resistance to a second herbicide, wherein each of the regions is under expression control of a plant operable promoter and terminator wherein the first herbicide is a post emergence herbicide and the second herbicide is a pre-emergence herbicide, wherein the proteins are selected from the group consisting of 5-enolpyruvyl-3-phosphoshikimate synthetase and HPPD.

2. Plants which comprise the polynucleotide of Claim 1.
